# EUROPÄISCHE PATENTSCHRIFT

(11) **EP 2 199 447 B1**
(45) Veröffentlichungstag und Bekanntmachung des Hinweises auf die Patenterteilung: **01.08.2012**
(21) Anmeldenummer: 09002419.1
(22) Anmeldetag: 20.02.2009
(51) Int. Cl.: D04H 13/00, B32B 5/06, B32B 27/12, A61L 15/60, A61F 13/15

(54) **Superabsorberpolymerfilz und Verfahren zu dessen Herstellung**
Superabsorbent polymer felt and method for its manufacture
Feutre polymère super-absorbant et son procédé de fabrication

(30) Priorität: 19.12.2008 DE 102008063229
(43) Veröffentlichungstag der Anmeldung: 23.06.2010
(73) Patentinhaber: Dehn, Michael C., 20249 Hamburg (DE)
(72) Erfinder: Dehn, Michael C., 20249 Hamburg (DE)
(74) Vertreter: Kossak, Sabine

(56) Entgegenhaltungen:
- EP-A- 0 278 419
- EP-A- 0 491 454
- WO-A-00/04936
- WO-A-91/01766
- WO-A-2004/016425
- CH-A5- 629 137
- US-A- 5 296 290

## Beschreibung

Gegenstand der vorliegenden Erfindung ist ein Filzmaterial mit Sperrfunktion umfassend mindestens eine Filzschicht und ein absorberhaltiges Material sowie ein Verfahren zur Herstellung des Filzmaterials, ein Bauteil aus diesem Filzmaterial und dessen Verwendung.

Filz ist ein textiles Flächengebilde, das aus mechanisch verfestigten Fasern besteht. Anders als andere Flächengebilde ist Filz nicht gewebt, sondern ein durch die Walkprozedur unter Einwirkung von Druck und feuchter Wärme verfestigtes Vlies. Traditionell werden Filze aus Wolle hergestellt oder aus anderen losen Tierhaaren, da es durch die Schuppenstruktur der Haare zu einer Verhakung der Fasern untereinander kommt. Dabei kann Filz in Härten zwischen watteweich und holzhart oder sogar härter produziert werden. Die klassischen Wollfilze werden auch als Walkfilze bezeichnet.

In jüngerer Zeit werden Filze zunehmend als Nadelfilz hergestellt. Hierbei werden Fasern übereinander geschichtet und mit zahlreichen Nadeln, die mit Widerhaken versehen sind, mehrfach durchstochen. Durch das wiederholte Einstechen werden die Fasern miteinander verschlungen bzw. in den Filz gedrückt. Die Nadelfilze lassen sich deshalb nicht nur aus Wolle, sondern aus praktisch allen bekannten Fasern herstellen.

Filz wird aufgrund seiner wärmenden Eigenschaften sowohl für die Herstellung von Bekleidung, Futterstoffen oder Schuhen, als auch für Dämmmaterialien verwendet. Besonderer Beliebtheit erfreuen sich wegen des wärmenden Effektes, beispielsweise Schuhe aus Filz, Janker oder Filzhüte. In der Technik werden beispielsweise Dichtungen, wie Filzringe oder Filzstreifen oder flächige Materialien zur Isolierung oder Geräuschdämmung aus Filz eingesetzt. Zudem sind auch technische Formteile oder Filter aus Filzmaterial bekannt. Filz ist neben seinen wärmedämmenden Eigenschaften luftdurchlässig und im gewissen Umfang wasserabweisend. Bei einer größeren Menge Wasser, beispielsweise durch Regen, dringt das Wasser jedoch durch den Filz hindurch. Der Filzstoff fühlt sich dann unangenehm nass und schwer an.

Im Bereich von Hygieneartikeln oder bei Polsterstoffen ist es bekannt, Schichtmaterialien mit Superabsorberpolymeren zur Aufnahme von Wasser und Feuchtigkeit einzusetzen. So beschriebt die DE 698 33 007 T2 ein absorbierendes Produkt aus mehreren Schichten, das einen absorbierenden Kern zur Aufnahme von Flüssigkeit aufweist. Zum Schutz der Bekleidung ist eine der Schichten dabei aus Kunststoffmaterial gefertigt, so dass die Luftdurchlässigkeit des Produktes stark eingeschränkt ist. Die eingesetzten Produkte zudem so konfektioniert, dass sie eine maximale Flüssigkeitsaufnahme bieten. Die hierbei entstehende deutliche Gewichtszunahme und Volumenzunahme des Produktes sind für den Anwendungsbereich Hygieneartikel bisher nicht kontrollierbar. Zudem sind die Produkte nur zur Einmalanwendung geeignet und müssen nach dem Flüssigkeitskontakt entsorgt werden. Die Produkte sind zudem nicht waschbar und der Superabsorber würde sich durch mechanische Einflüsse, wie bei der Maschinenwäsche oder bei dauerhaftem Zusammenpressen, vom Trägermaterial ablösen.

Für Hygieneartikel ist es weiter aus der DE 10 2007 016 959 bekannt, Faserstoffbahnen aus Zellstofffasern herzustellen und die Absorbtion dieser zellstofffaserhaltigen Schichten durch Zugabe eines Superabsorbers zu verbessern. Auch die hier beschriebenen Produkte sind für Einmalartikel, wie Hygieneartikel, Krankenauflagen, Windeln, Slipeinlagen, Einlage von Lebensmittelverpackungen oder einmalige Filter, einsetzbar. Eine Verwendung der beschriebenen Materialien in Kleidungsstücken oder Bereichen, wo ein mehrmaliger Einsatz notwendig wäre, ist durch die geringe mechanische Festigkeit und die hohe Aufquellung bei Flüssigkeitskontakt nicht möglich. Aufgrund der großen Menge Flüssigkeit, die pro Fläche aufgenommen wird, trocknen diese Materialien nur langsam ab, da ein großes Flüssigkeitsvolumen verdampfen muss. Der Artikel hat zudem durch die große Flüssigkeitsaufnahme ein deutliches Gewicht.

Aufgabe der vorliegenden Erfindung ist es daher, ein absorberhaltiges Material bereitzustellen, das eine hohe mechanische Festigkeit besitzt und im trocknen Zustand eine gute Luftdurchlässigkeit aufweist. Das absorberhaltige Material soll gleichzeitig im nassen oder feuchten Zustand eine Sperrwirkung gegen Flüssigkeits- oder Feuchtigkeitseintritt aufweisen. Die mit dem Material hergestellten Gegenstände sollen waschbar oder chemisch zu reinigen sein. Weiterhin soll das absorberhaltige Material zur Klimaregulierung, wie z.B. der Belüftung, Entfeuchtung und/oder Befeuchtung, von damit ausgerüsteten Gegenständen geeignet sein.

Weiterhin ist Aufgabe der Erfindung ein einfaches und kostengünstiges Verfahren zur Herstellung eines solchen Filzmaterials bereitzustellen, das möglichst ohne Verwendung spezieller Maschinen durchgeführt werden kann.

Die Aufgabe wird erfindungsgemäß gelöst durch ein Filzmaterial mit Sperrfunktion zur Klimaregulierung umfassend mindestens zwei Filzschichten und mindestens in Teilbereichen mindestens eine flüssigkeitaufnehmende Schicht, wobei
- die mindestens eine flüssigkeitaufnehmende Schicht eine absorberhaltige Schicht ist,
- die erste Schicht eine Filzschicht ist mit einer zumindest in Teilbereichen darauf angeordneten absorberhaltigen Schicht und einer weiteren darauf angeordneten zweiten Filzschicht,
- die mindestens zwei Filzschichten und die absorberhaltige Schicht (3) miteinander verfilzt sind,
- der Absorber in seiner dreidimensionalen Ausdehnung durch die Filzschichten und ggf. die Sperrelemente begrenzt ist,
- das Filzmaterial im trockenen, geöffneten Zustand zumindest teilweise luftdurchlässig ist und
- das Filzmaterial bei Kontakt mit Flüssigkeit, Wasser oder Wasserdampf sich durch die Ausdehnung des Absorbers verschließt, wobei der Flüssigkeitstransport durch das Filzmaterial durch den gequollen Absorber begrenzt oder gestoppt wird,
   dadurch gekennzeichnet, dass
   die Absorberschicht ein Absorbervlies, ein Absorberpolymer, ein Absorber mit Trägermaterial oder Absorberfasern, und
   der Absorber
   - ein Superabsorberpolymer oder
   - ein quellfähiges Polymer, ausgewählt aus der Gruppe bestehend aus Polyacrylsäure, Polyacrylsäurecopolymeren, vernetzten Natriumpolyacrylat, Casein, Eiweiß und Thermoplast-Elastomer-Compositen oder
   - eine Superabsorberpolymerfaser
      ist,
      der Absorber in seiner dreidimensionalen Ausdehnung zumindest teilweise durch die Verbindungsfasern zwischen den Filzschichten begrenzt ist und das Filzmaterial (1) ein Nadelfilz ist.

Weiterhin wird die Aufgabe gelöst durch ein
Verfahren zur Herstellung eines Filzmaterials umfassend mindestens zwei Filzschichten und zumindest teilweise mindestens eine absorberhaltige Schicht,
wobei der Absorber bei Kontakt mit Flüssigkeit, Wasser oder Wasserdampf aufquillt, dadurch gekennzeichnet, dass
(a.1)- die mindestens eine absorberhaltige Schicht auf eine Filzschicht oder zwischen zwei Filzschichten platziert wird und
(a.2)- die Filzschichten und die absorberhaltige Schicht mit Nadeln miteinander verfilzt werden, wobei die absorberhaltige Schicht ein Absorbervlies ist, wobei die Fasern der einen Filzschicht die weitere Filzschicht durchdringen und Verbindungsfasern bilden
   oder
(b.1)- eine Schicht mit Vorprodukten des Absorberpolymers auf eine erste Filzschicht oder zwischen zwei Filzschichten platziert wird und
(b.2)- die Filzschichten und die vorproduktehaltige Schicht mit Nadeln miteinander verfilzt werden und die Polymerisation zum Absorberpolymer während oder nach dem Filzvorgang abläuft und beendet wird,
   wobei die Fasern der einen Filzschicht die weitere Filzschicht durchdringen und Verbindungsfasern bilden.

Weitere Ausführungsformen sind nachfolgende beschrieben oder Gegenstand der Unteransprüche.

Das erfindungsgemäße Filzmaterial ist im trocknen Zustand luftdurchlässig, so dass eine Belüftung der mit dem Filzmaterial versehenen Bekleidungsstücke, Schuhe oder Gegenstände erfolgt. Sobald das erfindungsgemäße Filzmaterial mit Flüssigkeit, wie z.B. Wasser oder mit Wasserdampf (Feuchtigkeit) in Berührung kommt, quillt der eingeschlossene Absorber auf und begrenzt so den Durchfluss durch das Filzmaterial. In diesem als nassen Zustand bezeichneten Zustand ist das Filzmaterial bevorzugt weder für Luft noch für Wasserdampf noch für Flüssigkeiten durchlässig, d. h. es weist eine Sperrfunktion für Wasser/Flüssigkeit auf. Unter Wasser wird hierbei Wasser im flüssigen Aggregatzustand verstanden, während unter Wasserdampf (Feuchtigkeit) gasförmiges Wasser verstanden wird. Ebenso wie mit Wasser quillt der Absorber auch bei Berührung mit anderen Flüssigkeiten auf, wobei es quellverursachende und inerte Flüssigkeiten gibt. Die Berührung mit inerten Flüssigkeiten führt nicht zu einer Quellung. Die verschiedenen Absorberpolymere reagieren dabei unterschiedlich auf Wasser bzw. bestimmte Flüssigkeiten. Ein Verschließen des Filzmaterials und somit eine Aktivierung der Sperrfunktion erfolgt bevorzugt durch Berührung mit Wasser. Eine Berührung mit Wasserdampf führt entweder zu einer leichten Quellung oder bei hoher Absorberdichte zu einem Verschließen des Materials.

Das erfindungsgemäße Filzmaterial hat den Vorteil, dass es waschbar ist, beispielsweise beim Einsatz in Bekleidungsstücken sogar einer normalen Maschinenwäsche unterzogen werden kann und hierdurch einen breiten Einsatz in verschiedensten Bereichen ermöglicht. Durch die Waschbarkeit wird für viele Bereiche erstmals eine Wiederverwendbarkeit der mit Absorberpolymer versehenen Anwendungsprodukte erreicht. Hierdurch lässt sich der beim Einsatz von Einwegprodukten anfallende Müll deutlich reduzieren.

Da das erfindungsgemäß Filzmaterial in verschiedenen Ausführungsformen die gleichen Eigenschaften wie ein entsprechendes nicht mit Absorberpolymeren versehenes Filzmaterial aufweist, ist es möglich, das erfindungsgemäße Filzmaterial überall dort einzusetzen, wo auch normales Filzmaterial eingesetzt wird. Im trockenen Zustand ist das erfindungsgemäße Filzmaterial sowohl optisch als auch haptisch nicht von normalem Nadelfilz zu unterscheiden. Hinsichtlich des Aussehens und des Anfassens wird somit ein zum herkömmlichen Nadelfilz gleichwertiges Material erhalten. Im Vergleich zu herkömmlichen absorberhaltigen Flächenmaterialien, zeigt das erfindungsgemäße Filzmaterial jedoch eine höhere mechanische Strapazierfähigkeit und eine höhere Stabilität.

Überraschenderweise lässt sich das erfindungsgemäße Filzmaterial auf den gleichen Maschinen wie Nadelfilz herstellen, ohne dass es hierfür nötig ist, eine besondere Einstellung oder Umstellung des Filzvorgangs vorzunehmen. Durch die Herstellung entstehen somit keine höheren Verarbeitungskosten als bei der Herstellung eines normalen Filzes.

Gegenüber herkömmlichen Materialien, die mit Superabsorberpolymeren versehen sind, wie beispielsweise die aus dem Stand der Technik bekannten Inkontinenzprodukte, weist das erfindungsgemäße Filzmaterial den Vorteil auf, dass die Superabsorberpolymere unverlierbar eingeschlossen werden, d.h. bei Gebrauch des erfindungsgemäßen Filzmaterials nicht ungewollt Teile des Absorberpolymers aus dem Filzmaterial austreten können und hierdurch die Anwendungseigenschaften beeinträchtigen.

Ein weiterer Vorteil des erfindungsgemäßen Filzmaterials und des aus diesem Filzmaterial hergestellten Bauteils besteht darin, dass das Material besonders leicht zu konfektionieren und auch hierdurch leicht an verschiedenste Anwendungsgebiete anzupassen ist. Das erfindungsgemäße Filzmaterial weist zudem eine große Formbarkeit auf, so dass gezielt bestimmte Formen und Formteile hergestellt werden können.

Insbesondere durch die Waschbarkeit und die hohe Stabilität hat das erfindungsgemäße Filzmaterial eine deutlich höhere Lebensdauer als andere mit Superabsorberpolymeren versehene Funktionsrnaterialien.

Das erfindungsgemäße Material unterscheidet sich in seiner Luftdurchlässigkeit wesentlich von den bekannten Klimamembranen, die nur in eine Richtung und nur für Wasserdampf durchlässig sind. Während bei dem erfindungsgemäßen Filzmaterial ein Umschalten zwischen durchlässigem und nicht durchlässigem Zustand möglich ist und Luft sowohl von innen nach außen als auch von außen nach innen transportiert wird, ermöglichen die Klimamembranen nur einen Transport von Wasserdampf in eine Membranrichtung. Ein Transport von Luft erfolgt durch die Klimamembranen nicht. Dieses stellt einen der wesentlichen Vorteile des erfindungsgemäßen Materials gegenüber Klimamembranen dar.

Das Verschließen des Filzmaterials erfolgt durch die Begrenzung des Absorbers in seiner dreidimensionalen Ausdehnung. In einer Ebene ist der Absorber durch die Schichten des Filzmaterials in seiner Ausdehnung begrenzt. In den beiden anderen Ebenen wird der Absorber durch die Fasern, die die beiden Filzschichten verbinden und hierdurch kleine kammerartige Räume bilden, in seiner Ausdehnung begrenzt sind. Diese Fasern werden erfindungsgemäß als Verbindungsfasern bezeichnet. Durch den Einschluss des Absorbers im Filzmaterial wird ein absorberhaltiges Material mit einer hohen mechanischen Festigkeit erhalten. Dieses stellt einen besonderen Vorteil gegenüber bekannten absorberhaftigen Artikeln dar, da er es ermöglicht, das erfindungsgemäße Filzmaterial auch in Bereichen einzusetzen, bei denen eine hohe mechanische Belastung erfolgt oder bei denen ein mehrfacher Einsatz gewünscht ist.

Die erste und die zweite Filzschicht werden bevorzugt so miteinander verbunden, dass der Absorber möglichst gleichmäßig verteilt und von den Verbindungsfasern unverlierbar umschlossen wird. Die dreidimensionale Ausdehnung wird somit hauptsächlich durch die Verbindungsfasern zwischen den Filzschichten sowie ggf. das Trägermaterial begrenzt.

In einer weiteren Ausführungsform kann die Begrenzung zusätzlich zu den Fasern auch durch weitere Strukturen in den Randbereichen, wie Kunststoffumrandungen, erfolgen.

Das erfindungsgemäße Filzmaterial besteht aus mindestens drei Schichten, wobei die erste Schicht ein Filzmaterial ist, auf der eine zweite Schicht angeordnet ist aus einem absorberhaltigen Material und auf der absorberhaltigen Schicht eine weitere zweite Filzschicht angeordnet ist. Die beiden Filzschichten bestehen entweder aus demselben Fasermaterial oder aus unterschiedlichen Fasermaterialien.

### Filzschicht

Die Filzschicht des erfindungsgemäßen Filzmaterials bestehen bevorzugt aus synthetischem, halbsynthetischen, tierischen, mineralischen, metallischen, pflanzlichen Fasern, Gummifasern, Hybridfasern oder einer Mischung hiervon. Geeignete Fasern sind beispielsweise Wollfasern, Carbonfasem, Mikrofasern oder synthetische Fasern aus Polypropylen (PP) oder Polyester (PES). Die Fasern haben je nach Ausführungsform alle den selben Durchmesser oder zumindest teilweise unterschiedliche Durchmesser. Als Hybridfasern werden Fasern bezeichnet, die aus mehr als einem Ausgangsmaterial, beispielsweise verschiedenen Kunststoffen bestehen.

In einer Ausführungsform ist die Filzschicht des erfindungsgemäßen Filzmaterials ganz oder teilweise aus nachwachsenden Rohstoffen hergestellt. Fasern solcher nachwachsenden Rohstoffe sind z. B. Polysaccharidfasern und insbesondere Stärke-, Zellulose-, Guaran-, Guar- und Peptinfasern oder Fasern aus deren Derivaten. Beim Einsatz von Fasern oder Faseranteilen aus nachwachsenden Rohstoffen, ist es möglich, das erfindungsgemäße Filzmaterial als biologisch abbaubares bzw. kompostierbares Material herzustellen.

Die Filzschicht des erfindungsgemäßen Filzmaterials kann aus einheitlichen Fasern oder einer Fasermischung bestehen. Bei der Verwendung von Fasergemischen ist es möglich, Fasern mit unterschiedlichen Eigenschaften zu einem Filz zu verarbeiten. Die Fasern des Fasergemisches können sich dabei z.B. in der Faserdicke, der Faserdichte oder der Oberflächenstruktur unterscheiden. Es können beispielsweise Fasern mit einer Oberflächenstruktur verwendet werden, welche eine Verfilzung unterstützen, wie dieses bei tierischen Fasern z.B. Wollfasern mit Schuppen der Fall ist. Die Fasern können hierzu Ein- und Auswölbungen oder andere Oberflächenstrukturen aufweisen. Beim Einsatz von Fasergemischen sind die unterschiedlichen Fasern entweder gleichmäßig oder ungleichmäßig im Faserflächegebilde verteilt, bevor sie zum Filz weiterverarbeitet werden.

In einer Ausführungsform ist das Fasergemisch so ausgestaltet, dass es einen Faseranteil enthält, der besonders gut von den Filznadeln erfasst wird und einem zweiten Faseranteil, welcher eine spätere Weiterverarbeitung des Filz unterstützt. Ein solches zweites Fasermaterial kann beispielsweise aus schmelzbaren Fasern aufgebaut baut sein, so dass der Filz durch thermisches Pressen, Verdichten, Glätten oder Strukturieren verformen lasst. Weiterhin ist es möglich, dass z.B. der zweite Faseranteil mit einer chemischen Komponente behandelt ist, die im späteren Herstellungsprozess beispielsweise durch UV-Bestrahlung, Wärmeaushärtung oder chemische Reaktion weiterverarbeitet werden kann.

In weiteren Ausführungsformen ist die Filzschicht ganz oder zumindest teilweise aus Hohlfasern oder Funktionsfasern bestehend. Solche Funktionsfasern sind beispielsweise mit einem Latentwärmespeicher, wie z.B. einen Phasenwechselmaterial PCM, das unter der Marke Micronal^{®} PCM von der BASF angeboten wird, ausgerüstet oder enthalten in verkapselter Form Duft-, Wirk- und Farbstoffe. Als Funktionsfaser kommen weiterhin Metallfasern, wie z.B. Silberfasern oder mit Silberionen ausgerüstete Fasern, ebenso in Frage, wie Fasern mit schmelzbaren Kunststoffen oder schmelzbaren Oberflächenüberzügen. Je nach Einsatzbereich können die entsprechenden Filzfasem auch aus schwer entflammbaren Materialien bestehen. Weitere geeignete Funktionsfasern sind hitzebeständige Fasern, wie z.B. Carbonfasern und hochfeste Fasern, wie z.B. Aramidfasern. Zudem können als Funktionsfasern mit Superabsorberpolymer versehene Fasern verwendet werden. Die Funktionsfasern können dabei so ausgerüstet sein, dass die Fasern selektiv auf Temperatur, Feuchtigkeitsgehalt, pH-Wert, Stromspannung oder Druck reagieren und in Abhängigkeit von diesem Parameter ein unterschiedliches Verhalten zeigen. Ebenso können die Fasern so ausgerüstet sein, dass sie selektiv bestimmte chemische Stoffe binden oder selektiv bestimmte Stoffe abgeben. Die Fasern können zudem in einer Ausführung antistatisch und somit staubabweisend ausgerüstet sein.

Die Filzschicht des erfindungsgemäßen Filzmaterials ist in einer weiteren Ausführung mit zusätzlichen feuchtigkeitsleitenden Materialien ganz oder teilweise umhüllt. Hierdurch wird eine Kapillarwirkung, die die Feuchtigkeit in oder aus der absorberhaltigen Schicht leitet, erreicht. Alternativ ist bei dieser Ausführung an Stelle der Umhüllung ein Zusatzmaterial Bestandteil des Filz, das beispielsweise Cellulosefasern, Flocken, Formkörper oder ein Pulver ist und durch seine Kapillarwirkung ebenfalls Wasser bzw. Flüssigkeit aus oder in die absorberhaltige Schicht leitet.

Weitere geeignete Funktionsfasern sind z.B. lichtleitende oder lichtspeichernde Fasern, wie z.B. polymeroptische Fasern (POF).

In einer weiteren Variante sind die Fasern der Filzschicht mit weiteren Stoffen versehen, wie z.B. Farbstoffen, Duftstoffen oder Hilfsstoffen. Als Hilfsstoff können beispielsweise Materialien zur wasserabweisenden Ausgestaltung der Oberfläche, zur flammenhemmenden Ausgestaltung der Oberfläche oder zur Erzeugung bestimmter Eigenschaften der Stoffe erreicht werden. Ein weiterer Hilfsstoff, der beispielsweise auf die Fasern der Filzschicht oder zwischen die Filzschichten eingebracht sein kann, ist ein geruchsbindendes Material bzw. eine geruchsbindende Substanz, wie z.B. Metallsalze der Rezinolsäure, Aminosäuren oder andere Geruchsbindner. Diese werden beispielsweise auch in verkapselter Form eingesetzt, so dass sie nach und nach von dem Material freigesetzt werden.

In einer Ausführung werden die Duftstoffe, Farbstoffe und/oder Wirkstoffe nachträglich auf das erfindungsgemäße Filzmaterial aufgebracht. So kann z.B. Filzmaterial nachträglich mit Duftstoffen oder Farbstoffen versehen werden, damit es individuell gestaltet werden kann. Geeignete Wirkstoffe sind beispielsweise geruchsbindende Stoffe, mikrobiozid wirksame Stoffe oder Insekten vertreibende Stoffe. Als eine Art von Wirkstoff kann das Material mit Indikatorstoffen versehen sein, die anzeigen wenn der Belüftungseinsatz mit Substanzen in Berührung gekommen ist, die z.B. die Funktionsfähigkeit einschränken oder zerstören. Diese Anzeige kann beispielsweise durch einen Farbumschlag erfolgen. Als ein Wirkstoff können Substanzen zugesetzt sein welche die Trocknung des Absorbers beschleunigen wie etwa Materialien, welche dem Absorber Feuchtigkeit entziehen oder durch kinetische Energie Strom oder chemische Reaktion Wärme erzeugen. Es ist auch denkbar das Filzmaterial beheizbar auszuführen und hierdurch eine beschleunigte Trocknung des Absorbers zu erreichen.

In einer weiteren Ausführungsform ist das erfindungsgemäße Filzmaterial mit Hilfsstoffen versehen, die die Anwendungseigenschaften für die jeweilige Anwendung verbessern. So ist in einer Variante das erfindungsgemäße Filzmaterial beispielsweise mit Hautpflegestoffen oder wundheilfördernden Materialien beschlagen. Geeignete Hautpflegestoffe oder wundheilfördernde Materialien sind beispielsweise Dexpanthenol, Hamamelis, Kamille, Antioxidantien, Lichtschutzmittel, Hyaloronsäure, Insektenrepellentien, antimikrobielle Substanzen, ätherische Öle, Moisturizer oder Parfüm. Die aufgegebenen vorgenannten Zusatzstoffe werden dem erfindungsgemäßen Filzmaterial direkt oder in verkapselter Form zugesetzt oder sind Bestandteil der Fasern oder des Absorberpolymers.

In einer weiteren Variante ist die Filzschicht des erfindungsgemäßen Filzmaterials mehrschichtig aufgebaut, wobei die verschiedenen Schichten aus unterschiedlichen Fasern oder Materialien bestehen können. So kann beispielsweise eine gewebte Schicht (woven) mit einer weiteren Schicht aus filzbaren Fasern verbunden sein. Auch die Verbindung einer Kunststoffschicht mit einer filzbaren Faserschicht kann erfindungsgemäß eingesetzt werden. In einer Ausführungsform enthält die Filzschicht des erfindungsgemäßen Filtermaterials Beimischungen. Geeignete Beispielmischungen sind beispielsweise die bereits beschriebenen Funktionsfasern, wie z.B. PCM, Keramikfasern, Glasfasern, Carbonfasern, Absorberpolymerfasern und/oder metallische Fasern. Durch die Beimischung entsprechender Fasermaterialien lassen sich unterschiedliche Materialeigenschaften und unterschiedliche Flexibilitäten der Materialien einstellen.

Wenn das erfindungsgemäße Filzmaterial aus einer ersten Filzschicht, der darauf angeordneten absorberhaltigen Schicht und einer zweiten Filzschicht aufgebaut ist, sind die erste und zweite Filzschicht entweder aus dem gleichen Fasermaterial oder unterschiedlich beschaffen. Die Beschaffenheit der Filzschicht hängt dabei vom jeweiligen Anwendungsbereich ab. So ist es beim Einsatz des erfindungsgemäßen Filzmaterials für Filter beispielsweise sinnvoll, eine Seite mit Carbonfasern zu versetzen, um eine Filterwirkung zu erreichen, bevor Luft oder Flüssigkeiten, die nicht zu einer Quellung führen auf die absorberhaltige Schicht treffen. In anderen Bereichen kann einer der beiden Filzschichten beispielsweise so ausgerichtet sein, dass sie über wasserabperlende Eigenschaften verfügt. In einer weiteren Variante ist eine der beiden Filzschichten so mit Duft-, Wirk- oder Farbstoffen versehen, dass die durchströmende Luft oder die nicht zur Quellung anregende Flüssigkeit beim Verlassen des Filzmaterials mit den Duft-, Wirk- oder Farbstoffen beaufschlagt wird.

### Absorberhaltige Schicht

Die absorberhaltige Schicht enthält mindestens einen Absorber bevorzugt einen auf einem Trägermaterial aufgebrachten Absorber. Bevorzugt ist die absorberhaltige Schicht des erfindungsgemäßen Filzmaterials ein Absorbervlies, besonders bevorzugt ein Vlies mit Absorberpolymeren. Der Absorber ist dabei Superabsorberpolymer, d.h. ein bei Berührung mit Flüssigkeit, insbesondere Wasser, quellfähiges Polymer, bevorzugt ausgewählt aus der Gruppe bestehend aus Polyacrylsäure, Polyacrylsäurecopolymeren, vernetzten Natriumpoylacrylat, Casein, Eiweiß und Thermoplast-Elastomer-Kompositen. Besonders bevorzugt wird als Absorbervlies, Luquafleece^{®} von der BASF eingesetzt. Ein weiteres geeignetes Fleece, das mit Superabsorberpolymeren versehen ist, wird unter dem Namen Oasis^{®} von der Firma Technical Absorbants angeboten. Als Absorberpolymere können beispielsweise Superabsorber verwendet werden, wie HySorb^{®} von der BASF AG oder Favor^{®} von der Degussa AG. Optional ist der Absorber mit einem Füllstoff vermischt und Füllstoff und Absorber bilden gemeinsam die absorberhaltige Schicht aus. Ebenso können z.B. absorberpolymerhaltige Flächen, wie sie in der Hygiene-Industrie genutzt werden, als absorberhaltige Schicht verwendet werden.

In einer alternativen Ausführungsform enthält die absorberhaltige Schicht als Trägermaterial Fasern, an denen der Absorber gebunden ist, die sich in wässrigen oder nicht wässrigen Medien auflösen. Diese temporäre absorberhaltige Schicht wird ebenfalls zwischen die Filzschichten eingebracht, wobei jedoch nach dem Vernadeln die Fasern der absorberhaltigen Schicht aufgelöst werden, so dass nur der nicht mehr fasergebundene Absorber ohne Trägermaterial zwischen den Filzschichten verbleibt. Das Trägermaterial in Form von löslichen Stoffen oder löslichen Fasern kann z.B. durch Ausspülen, durch Druckluft, Hitze oder chemisch entfernt werden. Durch die Entfernung des Trägermaterials werden durchlässige Zwischenräume geschaffen, die eine verbesserte Luftdurchlässigkeit ergeben. Je nach dem Grad der Verdichtung des Filzmaterials ist der Absorber von den Verbindungsfasern und den nicht löslichen Anteilen des Trägermaterials mehr oder weniger fest umschlossen.

Die Auswahl des Absorbers hängt davon ab, durch welche Flüssigkeit die Quellung des Absorbers erfolgen soll. Bei einem Einsatz für die Quellung von Wasser werden bevorzugt die genannten Superabsorber verwendet. In einer Ausführung ist es auch möglich zwei oder mehrere Absorbermaterialien zu kombinieren, so dass ein Absorber auf Wasser und ein anderes Absorbermaterial auf eine andere Flüssigkeit anspricht.

Die Formgestaltung des Absorberpolymers wird in ihrer Größe bevorzugt angepasst, um zu gewährleisten, dass beim Verfilzen die Absorberpolymerpartikel oder -fasern im Wesentlichen zwischen den Filzschichten und/oder Verbindungsfasern angeordnet bleiben und nicht zusammen mit den Fasern neu angeordnet werden.

Als Füllstoff können beispielsweise eingesetzt werden Polymerverbindungen, Thermoplast-Elastomer-Composite, tierische Fasern, wie Haare, Daunen, Leder, Knochen, Hörn, pflanzliche Fasern wie Baumwolle, Cellulose, Pappe, Leinen, Kokosschalen, Holz, Faser, Kräuter-, metallische-, mineralische Fasern, Carbonfaser-Gewirke, Gummi bzw. Materialien oder Mischungen hieraus. Pulver oder Granulate oder Materialien mit wärmespeichernden Eigenschaften z.B. mikroverkapselte Paraffinwachse (PCM). Das Füllmaterial dient z.B. dazu, das Gewicht zu reduzieren. Wird ein flüssigkeits- oder wasserleitendes Füllmaterial eingesetzt, kann dieses zudem die zuvor aufgenommene Flüssigkeit bzw. das Wasser im Absorbers durch Bildung einer gezielten Feuchtigkeitsbrücke nach außen leiten und so eine schnellere Verdunstung des Wassers herbeiführen. Das Füllmaterial und die Menge des Füllmaterials sind so gewählt, dass die Quellung des Absorbers und die hierdurch bedingte Schließung des Filzmaterials auf den Anwendungsbereich eingestellt sind. Dient das Füllmaterial zur Bildung einer gezielten Feuchtigkeitsbrücke, so steht es vorteilhafterweise mit Verdünstungsflächen am zu belüftenden Objekt in Kontakt. Solche Verdünstungsflächen können z.B. Obermaterialien eines Bekleidungsstücks oder eines Schuhs sein. Der Einsatz eines solchen Füllmaterials ist insbesondere dann sinnvoll, wenn das Obermaterial selbst keine Feuchtigkeitsbrücke zum Innenmaterial bilden kann und somit nur eine geringe Verdunstungsfläche zur Verfügung steht. Die absorberhaltige Schicht kann in einer Ausführungsform mit weiteren Funktionsstoffen, wie z. B Aktivkohle oder Silberionen vermischt sein.

In einer weiteren Variante besteht die absorberhaltige Schicht zumindest teilweise aus Superabsorberpolymerfasern.

Die absorberhaltige Schicht ist in einer weiteren Ausführungsform eine quellbare Folie, beispielsweise eine mit Superabsorberpolymeren versetzte Kunststofffolie aus einem thermoplastischen Polyurethan-Elastomer. Bei Verwendung dieser Folie werden beim Filzen Fasern der ersten Filzschicht und der zweiten Filzschicht durch die Folien hindurchgezogen, so dass Perforationen zum Luftdurchtritt in der Folie entstehen. Sofern in einer weiteren Variante die Filzschichten zusätzlich Superabsorberpolymerfasern enthalten, können die Perforationen durch die quellenden Superabsorberpolymerfasern verschlossen werden. Zudem ist bekannt, dass auch die Folienquellen, so dass sich, wenn auch mit geringerer Geschwindigkeit, bei Wasserkontakt der Durchmesser der Perforationen verringert und den Verschluss unterstützt.

In einer weiteren Ausführungsform besteht die absorberhaltige Schicht teilweise oder ganz aus nachwachsenden Rohstoffen. Das Trägermaterial, auf dem beispielsweise die Absorberpolymere angeordnet sind, besteht dabei aus biologisch abbaubaren Fasern, wie z.B. Polysaccharidfasern, insbesondere Stärke-, Cellulose-, Guaran-, Guar- oder Peptinfasern. Wenn das erfindungsgemäße Filzmaterial eine absorberhaltige Schicht aus nachwachsenden Rohstoffen, die biologisch abbaubar sind aufweist und gleichzeitig die Filzschichten ebenfalls aus einem biologisch abbaubaren Material bestehen, ist somit das gesamte erfindungsgemäße Filzmaterial kompostierbar.

Abhängig von der verwendeten absorberhaltigen Schicht ist die Ausbreitung von Flüssigkeiten innerhalb der Fläche des erfindungsgemäßen Filzmaterials einstellbar. Die Ausbreitung hängt dabei z.B. von der Art, Partikelgröße, Trägermaterial und Vernetzung des Absorbers z.B. des Superabsorberpolymers ab. Die Durchtränkung des Filzmaterials wird neben der Wahl des Absorbermaterials auch durch Barrieren im Material beeinflusst. Eine Barriere wird z.B. auch durch stärkeres mechanisches Verfestigen oder durch die Art der Fasern des Trägermaterials oder der Filzschicht, die Beschaffenheit des Absorbers bestimmt. Zudem ist es denkbar, dass entsprechende Flächen derart gepresst werden, dass eine Ausbreitung einer fließfähigen Substanz gezielt reduziert oder begrenzt wird.

Die Flüssigkeit breitet sich auf Grund des Gelblocking nicht innerhalb der absorberhaltigen Schicht aus. Die Absorberpolymere quellen derart stark, dass kein ganzflächiger Nässetransport stattfinden kann. Wenn ein Flüssigkeitstransport erwünscht ist werden dem erfindungsgemäßen Filzmaterial deshalb hydrophile Materialien innerhalb oder verbunden mit der Filzschichteingesetzt. Hydrophile Materialien können z.B. Bestandteil des Absorber-Trägermaterials sein, als Ummantelung des reaktiven Materials, oder als Beimischung vorliegen, z.B. als Fasern, welche als Transportflächen Bestandteil des Filzes sind. Derzeit werden z.B. Zellulosefasern oder -partikel verwendet, um dieses Problem zu lösen. Alternativ werden Filzfasern genutzt die Transportfunktion zu übernehmen,. Die Transportmaterialien sind ggf. flächig derart gefilzt, dass sie nicht die Filzfläche durchdringen, sondern nur in die absorberhaltige Schicht ragen und Ober- und Unterseite bilden. Hierdurch ist ein gleichmäßiger Nässetransport gegeben. Der Nässetransport wird alternativ auch durch ein separates, mit dem Filz in Verbindung stehendes Material erreicht werden.

### Aufbau des erfindungsgemäßen Filzmaterials

Das erfindungsgemäße Filzmaterial ist aus mindestens zwei Filzschichten und zumindest teilweise einer absorberhaltigen Schicht, die miteinander vernadelt sind, aufgebaut.

In einer Ausführungsform ist das Filzmaterial aus mindestens drei Schichten aufgebaut:
- eine erste Filzschicht,
- eine zumindest in Teilbereichen auf der ersten Filzschicht angeordneten absorberhaltigen Schicht, beispielsweise aus einem auf ein Trägermaterial aufgebrachtes Absorberpolymer und
- einer zweiten Filzschicht, die auf der anderen Seite der absorberhaltigen Schicht für die erste Filzschicht angeordnet ist.

Solange das erfindungsgemäße Filzmaterial mindestens zwei Filzschichten und mindestens in Teilbereichen eine auf der ersten Filzschicht angeordnete absorberhaltige Schicht enthält, ist eine beliebige Kombination und Anzahl von absorberhaltigen Schichten und Filzschichten herstellbar. Die Anzahl der verwendeten Schichten hängt dabei von den gewünschten Materialeigenschaften und dem geplanten Anwendungsgebiet ab. Übliche Aufbauten sind beispielsweise drei Schichten. Aus erster Filzschicht, absorberhaltiger Schicht und zweiter Schicht, vier Schichten aus erster Filzschicht, absorberhaltiger Schicht, zweiter Filzschicht und Deckschicht und fünf Schichten aus erster Filzschicht, erster absorberhaltiger Schicht, zweiter Filzschicht, zweiter absorberhaltiger Schicht und dritter Filzschicht. Es ist beispielsweise auch möglich, zwei erfindungsgemäße Filzmaterialien mit verschiedenen Schichtaufbauten oder Materialeigenschaften durch eine weitere dritte Filzschicht miteinander zu verfilzen und zu kombinieren oder zwei erfindungsgemäße Filzschichten überlappend miteinander zu verfilzen.

Die Stärke der Verbindung zwischen erster Filzschicht, absorberhaltiger Schicht und zweiter Filzschicht wird durch die Durchdringungstiefe der Fasern eingestellt. Abhängig davon, ob die erste und zweite Filzschicht mechanisch miteinander verbunden sein sollen , wird eine entsprechende Durchdringungstiefe gewählt.
Die erste und zweite Filzschicht mit der dazwischen gelagerten absorberhaltigen Schicht werden dabei so miteinander verfilzt, dass eine zumindest teilweise oder großflächige Durchdringung der Materialien der beiden Filzschichten erreicht wird.

In einer Ausführungsform sind die erste und zweite Filzschicht so miteinander verfilzt, dass nur wenige direkte mechanische Verbindungen zwischen der ersten und der zweiten Filzschicht und somit der Ober- und Unterseite bestehen.

Diese Ausführungsform wird insbesondere eingesetzt, wenn Ober- und Unterseite des erfindungsgemäßen Filzmaterials unterschiedliche Eigenschaften haben. So ist es beispielsweise möglich, die Oberseite hydrophil auszurüsten, so dass diese ein Medium an den Funktionskern, die absorberhaltige Schicht, weiterleitet. Die Unterseite kann im Gegensatz dazu hydrophob ausgebildet sein und das Medium nicht an den Träger, z.B. des Kleidungsstücks, weiterleiten. Auch die umgekehrte Ausrüstung Unterseite hydrophil und Oberseite hydrophob ist eine mögliche Ausführungsform. Alternativ werden beispielsweise hydrophobe Fasern auf der Filzoberseite und der Filzunterseite verwendet, so dass Wasser durch die Faserzwischenräume dringen kann und stets zur absorberhaltigen Schicht geleitet wird und auch Wasserdampf über eine Distanz von dem Absorberpolymer aufgenommen wird. Beispielsweise ist das erfindungsgemäße Filzmaterial so aufgebaut, dass die eine Seite des Filzes aus Wolle oder Seide besteht, der Kern aus einem Superabsorberpolymer und die Unterseite aus Polyesterfasern.

Abhängig vom jeweiligen Anwendungsgebiet ist die absorberhaltige Schicht in Teilbereichen des Filzmaterials angeordnet, so dass das Filzmaterial in verschiedenen Bereichen unterschiedliche Eigenschaften aufweist. In diesem Fall ist die absorberhaltige Schicht bzw. das Superabsorberpolymer nicht vollflächig eingefilzt sondern nur in Teilbereichen, beispielsweise in Streifen, netzförmigen Anordnungen oder partiell eingearbeitet. Dieses führt dazu, dass die Teile, in denen sich keine absorberhaltige Schicht befindet, im nassen Zustand flexibler sind und so beispielsweise als Soll-Knickstellen genutzt werden können oder in Bereichen, in denen eine erhöhte Flexibilität notwendig ist, flexibler eingesetzt werden können. Alternativ ist die absorberhaltige Schicht im gesamten Filzmaterial angeordnet, wobei diese eine gleichmäßige oder unterschiedliche Dichte aufweist.

In einer Ausführungsform bildet die erste Filzschicht auf einer Seite gleichzeitig das Trägermaterial für das Absorberpolymer, beispielsweise indem die Filzschicht im Querschnitt mit nur zur Hälfte mit Absorberpolymer versehen ist und in der anderen Hälfte aus absorberfreiem Fasermaterial gebildet ist. Das erfindungsgemäße Filzmaterial wird in dieser Variante durch Umschlagen der Filzschicht, so dass die absorberhaltige Schicht in der Mitte zu Liegen kommt und anschließendes Verdichten (Verfilzen) der drei Schichten miteinander erhalten. Ein solches Material kann beispielsweise durch Auftragen von Absorberpolymeren auf ein Trägermaterial gebildet werden. In dieser Variante kann der Superabsorber auf beliebig dicke Faserschichten oder Filzschichten aufgebracht werden, wobei der Superabsorber vorteilhafterweise nur auf einer Seite aufgebracht ist.

Wenn die erste Filzschicht und die zweite Filzschicht und die absorberhaltige Schicht ggf. in Form von Absorbern auf einem Trägermaterial so angeordnet werden, dass sich die absorberhaltige Schicht mittig befindet, werden beim anschließenden Vernadeln die Fasern der beiden Filzschichten von den Filznadeln erfasst und durch den Filzaufbau gezogen. Die Absorberpolymere, die als Granulat stückig, in jedem Fall nicht als Faser, vorliegen, werden von den Filznadeln nicht erfasst, so dass die absorberhaltige Schicht auch nach dem Abschluss des Filzverfahrens im wesentlichen von den Filzschichten eingeschlossen ist.

In einer Variante ist die erste Filzschicht gleichzeitig das Trägermaterial für den Absorber, der Absorber ist dann Bestandteil der Filzschicht bzw. mit der Filzschicht verbunden oder auf dieser angeordnet.

Entsprechend enthält die Filzschicht je nach Ausführungsform einheitliche oder verschiedene Faserarten, auch innerhalb einer Textilbahn. Dabei unterscheiden sich die Faserarten beispielsweise in ihren Dichten (g/m²) und ihren Funktionen. Beispielsweise ist es so möglich, die Funktionsfasern nebeneinander oder sich teilweise oder ganz überlappend zu überfilzen.

In einer weiteren Ausführungsform enthält die absorberhaltige Schicht vor dem Filzen keinen Absorber, sondern besteht aus einem gewöhnlichen Material. Zumindest eine der beiden Filzschichten, entweder die erste oder die zweite Filzschicht oder beide Filzschichten, enthalten jedoch Superabsorberpolymerfasern, so dass während des Filzvorgangs die Superabsorberpolymerfasern zwischen die beiden Filzschichten gezogen werden. In diesem Fall sind die Filzfasern bevorzugt aus einem nicht quellbaren Material und bilden einen harten starren Filz. Die Superabsorberpolymerfasern werden in diesem Fall durch das harte Material des Filzes in ihrer Ausdehnung begrenzt. Optional ist das Filzmaterial direkt nach der Herstellung nicht luftdurchlässig, wobei durch die erste Quellung der Superabsorberpolymerfasern eine Verdrängung der Filzfasern erfolgt. Beim Schrumpfen der Absorberpolymerfasern werden dann Hohlräume geschaffen, durch die ein Luftdurchtritt erfolgen kann. Das Filzmaterial muss in diesem Fall durch eine erste Waschung zur vollen Funktionsfähigkeit aktiviert werden.

In einer weiteren Ausführungsform ist das erfindungsgemäße Filzmaterial so ausgebildet, dass zwischen den Filzschichten Zwischenräume, d.h. kleine Hohlräume, angeordnet sind, in denen ein stark quellender Absorber ausreichend Platz hat, um größere Mengen Flüssigkeit aufzunehmen und um bei Trockenheit die Luftdurchlässigkeit zu erhöhen. Die Zwischenräume verbessern zudem die Möglichkeit nachträglich einen Wirk-, Duft- oder Farbstoff in das Material, beispielsweise in die Zwischenräume, einzubringen. Die Zwischenräume können beispielsweise durch das im Vorhergehenden beschriebenen Verfahren Umfilzen eines löslichen Materials erreicht werden. Dieses lösliche Material dient als Platzhalter, so dass nach dem Ausspülen oder Herauslösen des Materials der beschrieben Zwischenraum entsteht.

In einer weiteren Variante werden die Hohlräume durch Formteile, z.B. aus Kunststoff oder aus Filz geschaffen. In diesem Fall erfolgt ein Verfilzen nur in Teilbereichen, so dass das erfindungsgemäße Filzmaterial über die gesamte Fläche eine unterschiedliche Filzstruktur aufweist. Das eingesetzte Formteil, beispielsweise in Form eines vorgefilzten Faserformteils oder eines Netzes wird zum einen zur Verstärkung der Stabilität oder zur Herstellung von Bauteilen mit bestimmten Umrissen eingesetzt. Zum anderen können die Formteile Hohlräume schaffen, die mit Absorber oder Absorbergranulat befüllt sind und ggf. zusätzlich zu den Verbindungsfasern beim Quellen des Absorbers zu dessen Verdichtung führen.

In einer Variante enthält das erfindungsgemäße Filzmaterial zusätzlich metallische oder formbare Fasern oder Streifen, entweder zwischen den Schichten oder als Teil der absorberhaltigen Schicht oder einer der Filzschichten. Ein entsprechend aufgebautes Filzmaterial wird beispielsweise für Anwendungen eingesetzt, bei denen herausragende Verdunstungsflächen vorhanden sind. Diese Verdunstungsflächen können dann so gebogen werden, dass eine möglichst optimale Flüssigkeitsaufnahme bzw. Abgabe erfolgt.

Eine Ausführungsform des erfindungsgemäßen Filzmaterials ist so gestaltet, dass die Oberfläche des Filzmaterials mit einer Struktur versehen ist. Die Struktur wird beispielsweise durch Verpressen oder Ausschneiden oder beim Filzvorgang auf das Material aufgetragen. In einer weiteren Variante wird die Oberflächenstruktur durch eine Deckschicht erzeugt. Durch eine strukturierte Oberfläche können Bereich mit unterschiedlicher Aufnahmefähigkeit für die Flüssigkeit sowie unterschiedlicher Abgabefähigkeit der aufgenommenen Flüssigkeit erzeugt werden. Bevorzugt ist die Oberflächenstruktur eine Rippenstruktur, die zu einer Vergrößerung der Oberfläche führt. Die Strukturierung der Oberfläche des erfindungsgemäßen Filzmaterials hat den Vorteil, dass die auftreffende Flüssigkeit bei großen Fließgeschwindigkeiten gebremst wird und so besser in das Innere des Filzmaterials zum Absorber geleitet werden kann. Die Erhebungen auf der Oberfläche des Filzmaterials führen dabei zu mechanischen Verwirbelungen und bremsen den Wasserstrom ähnlich wie ein Wellenbrecher. Die Erhebungen der Oberflächenstruktur können zudem als Abstandshalter dienen und hierzu beispielsweise mit einer nicht feuchtigkeitsaufnehmenden oder wasserdichten Deckschicht oder Oberflächenbehandlung versehen sein.

In einer weiteren Ausführungsform weist das erfindungsgemäße Filzmaterial Oberflächenstrukturen, Tunnelstrukturen oder Vertiefungen auf, um so die Verdunstungsfläche des erfindungsgemäßen Filzmaterials zu vergrößern. Die Strukturen sind dabei auf der Oberfläche oder im Filzmaterial oder sowohl auf der Oberfläche als auch im Inneren ausgebaut. Die inneren Strukturen können z.B. durch eingebrachte Bestandteile wie Wellpappe oder Kunststoffelemente erhalten werden. Die inneren Strukturen haben in einer weiteren Variante beispielweise eine wellenförmige Form. Durch Aufbringen einer ersten und zweiten Filzschicht auf den beiden Seiten der Welle entsteht eine der Wellpappe vergleichbare Struktur des erfindungsgemäßen Filzmaterials, bei dem Absorberpolymere beispielsweise in den Tälern der wellenförmigen Bahn, die in diesem Fall die absorberhaltige Schicht ist, angeordnet sein können.

Das erfindungsgemäße Filzmaterial weist in einer weiteren Ausführungsform Soll-Knickstellen bzw. Soll-Bruch- oder Abtrennstellen auf. Die Soll-Knick- bzw. Soll-Bruchstellen sind beispielsweise in Form von Schnitten, Lochungen, Vorstanzungen, Perforationen, Anschlitzungen, Anritzungen, Press-, Schmelz- oder Abtragvorgänge in das Material eingearbeitet. Die Einarbeitung von Soll-Knick- bzw. Soll-Bruchstellen führt bei der Verwendung von Hitze oder Ultraschall dazu, dass die entsprechenden Öffnungen versiegelt werden und somit ein Ausquellen der Absorberpolymerpartikel verhindert wird. Diese Ausführungsform erlaubt es dem Anwender das erfindungsgemäße Filzmaterial an eine Anwendungsgröße anzupassen, beispielsweise bei einem Dichtungsmaterial, ohne dass die Kanten nachträglich verschlossen werden müssen.

Das erfindungsgemäße Filzmaterial ist ggf. vorbehandelt, indem es zwei oder drei Mal durch Wasser bzw. Flüssigkeit aktiviert worden ist. Hierdurch kann für einzelne Anwendungsbereiche die Luftdurchlässigkeit und die Funktionsfähigkeit erhöht werden, da das Filzmaterial in diesem Falle "voraktiviert" ist.

In einer weiteren Ausführungsform ist das erfindungsgemäße Filzmaterial auf einer oder beiden Außenseiten mit einem luft- und/oder wasserundurchlässigen Material beschichtet. Das Filzmaterial weist dann gegebenenfalls Perforationen oder Öffnungen in der luft- und wasserundurchlässigen Schicht auf, bevorzugt auf der Oberseite und/oder Unterseite. Diese Perforationen oder Öffnungen sind wahlweise auf Ober-und Unterseite an den gleichen Stellen oder gegeneinander versetzt angeordnet. Die Perforationen und Öffnungen sind dabei entweder so ausgestaltet, dass sie jeweils nur einen Teil der Dicke bzw. Höhe der Filzschicht durchbrechen und nicht bis zur absorberhaltigen Schicht reichen oder bei Perforationen/Öffnungen, die kleiner oder nur wenig größer als der Absorber sind, bis in die absorberhaltige Schicht reichen. Die Öffnungen auf der Ober- und Unterseite bilden bevorzugt keine den Filz durchdringenden Öffnungen. Bevorzugt durchdringen die Perforationen oder Öffnungen die Filzschicht derart, dass noch eine die absorberhaltige Schicht haltende und/oder schützende Faserschicht als Abdeckung verbleibt. Die Ausführung des erfindungsgemäßen Filzmaterials mit Perforationen oder Öffnungen bietet einen Durchdringungsschutz, bei dem das Filzmaterial eine klimaregulierende Wirkung aufweist, gleichzeitig jedoch als Durchtrittschutz für andere Gase oder Flüssigkeiten verwendet werden soll. Die Perforationen und Öffnungen können beispielsweise durch Lasern, Brennen, Heißnadeln, Nadeln, mechanisches Abtragen, chemisches Abtragen, Luft oder Wasserstrahlschneiden, Ritzen, Bestrahlen unter Behandlung mit einem Gas, das eine kontrollierte Zersetzung ermöglicht, erfolgen. Zudem können die Perforationen oder Öffnungen auch durch Auflösen von Bestandteilen der Filzschicht, die als Platzhalter dienen, erfolgen, sofern diese Platzhalter mit Wasser oder einem geeignetem Lösemittel ausgespült werden oder mechanisch herausgelöst werden.

In einer weiteren Ausführungsform weist das erfindungsgemäße Filzmaterial Durchbrüche auf, die das erfindungsgemäße Filzmaterial teilweise oder vollständig im Querschnitt durchdringen. Diese Durchbrüche werden teilweise oder ganz durch Überspritzungen oder Laminat überdeckt. Das Laminat bzw. die Überspritzung weist bevorzugt Perforationen auf, durch die Wasserdampf aus dem erfindungsgemäßen Filzmaterial entweichen kann. Die abgedeckten Durchbrüche, d.h. die Vertiefungen bewirken beim Bewegen des Filzes eines Ventilation. Durch die Veränderung des Volumens der Durchbrüche bzw. Vertiefungen bei der Bewegung des Filzmaterials entsteht eine Luftzirkulation in den Vertiefungen. Die Luft kann dann durch die Öffnungen der Überspritzungen bzw. des Laminats abtransportiert werden, wodurch eine Kühlung, Belüftung, ein Abtransport von warmer Luft und eine Trocknung bewirkt wird. Die Öffnungen bzw. Perforationen im Laminat sind dabei entweder im Bereich der Durchbrüche bzw. Vertiefungen oder in Nachbarschaft zu diesen angeordnet. Abhängig von der Form der Vertiefungen bzw. Durchbrüche ist das erfindungsgemäße Filzmaterial einseitig oder beidseitig mit diesen Vertiefungen versehen. Wenn die Überspritzung bzw. das Laminat auf Höhe der Vertiefungen bzw. Durchbrüche des Filzmaterials angebracht ist, verhindert bzw. verzögert die Distanz zwischen Öffnung und Filz einen Wassertransport, wodurch eine Sättigung und die damit verbundenen Verschlusswirkung, d.h. Sperrfunktion, verhindert bzw. verzögert wird.

In einer weiteren Ausführungsform ist das erfindungsgemäße Filzmaterial ebenfalls mit Vertiefungen, Durchbrüchen oder Strukturen versehen, wobei das Laminat bzw. die Überspritzungen den Strukturen erfolgt. Wenn sich die Öffnungen bzw. Perforationen des Laminats oder der Durchspritzungen in denen Vertiefungen befinden, wird hierdurch vermieden, dass das Wasser direkt mit den Öffnungen in den Vertiefungen in Kontakt tritt. In diesem Falle würde das Wasser von der Oberfläche abperlen, Feuchtigkeit jedoch grundsätzlich durch die Öffnungen bzw. Perforationen aufgenommen oder abgegeben. Das Auftragen des Laminats, das Einbringen der Perforationen bzw. Öffnungen und das Strukturieren erfolgt beispielsweise in einem Arbeitsgang. Z.B. erfolgt das Pressen idealerweise im Zusammenhang mit Hitze, so dass sich das Laminat mit dem Filzmaterial verbindet und gleichzeitig die Strukturen ausgeformt und die Öffnungen bzw. Perforationen eingebracht werden.

Das erfindungsgemäße Filzmaterial ist in einer weiteren Ausgestaltung mit flächigen Strukturen, lamellen- oder schuppenförmigen Einschnitten versehen. Diese Strukturelemente sind so beschaffen, dass sie bei Aufnahme bzw. Abgabe von Wasser und/oder Flüssigkeit ihr Volumen verändern. Hierdurch heben oder senken sich die Strukturelemente aus der Oberfläche des erfindungsgemäßen Filzmaterials heraus. Entsprechend werden die Oberflächenänderungen beispielsweise als Designelement, um z.B. ein Logo oder einen Schriftzug erscheinen zu lassen, eingesetzt. Zudem ist es auch möglich, diese Designelemente als Indikator oder zur Veränderung der klimatisierenden Eigenschaften, beispielsweise der Verdunstungsfläche, einzusetzen. Sofern die Strukturelemente als Text oder Muster ausgestaltet sind, ist eine Gestaltung möglich, die es erlaubt, anhand der Anwesenheit oder Abwesenheit des Musters den Beladungszustand des erfindungsgemäßen Filzmaterials sichtbar zu machen.

Das erfindungsgemäße Filzmaterial hat weiter den Vorteil, dass auch andere Funktionsmaterialien als die Absorberpolymere besser in dem Filzmaterial gebunden sind als bei einem herkömmlichen Material. So werden beispielsweise PCM stärker in das Material eingebunden und verblieben deshalb länger im erfindungsgemäßen Filzmaterial als in üblichen mit PCM beschlagenem Materialien.

### Filzmaterial als Bauteil

In einer Ausführungsform ist das Filzmaterial als Meterware oder Plattenware ausgebildet und wird in dieser Form verwendet, beispielsweise als Dämmmatte oder für die jeweilige Anwendung als Bauteil konfektioniert. Das Filzmaterial wird in einer weiteren Ausführungsform direkt so hergestellt, dass es die für die Anwendung notwendige Form aufweist. In einer Ausführungsform ist das erfindungsgemäße Bauteil z.B. auch als dreidimensionaler Formkörper hergestellt.

In der Ausführung des Filzmaterials als Bauteil wird das Bauteil bevorzugt aus der Meter- oder Plattenware erhalten, das je nach Anwendung zugeschnitten, durch Pressen, z.B. mit Hitze und Druck, durch Formstanzen oder Lasern in Form gebracht wird. Die nach dem Schneiden offenen Schnittkanten, aus denen Absorber austreten könnten, werden ggf. vor dem Einsatz verschlossen, beispielsweise durch Nähte, Umketteln oder Auftragen eines zum Zeitpunkt der Verarbeitung fließfähigen Materials. Das Verschließen der offenen Schnittkanten durch Verarbeitung eines fließfähigen Materials kann beispielsweise durch Klebetechniken, wie im hot melt-Verfahren erfolgen. Das Verschließen der offenen Schnittkanten durch eine solche Randversiegelung ermöglicht den Einsatz von Filzmaterial in beliebiger Form für die verschiedenen Anwendungen. Bei einer großen Dichte der Verbindungsfasern wird ggf. auf Randversiegelung verzichtet. Alternativ können die Zuschnitte passgenau in die zu verschließende Öffnung oder eine passgenaue Halterung oder Rahmen eingesetzt werden. Durch das bündige Anliegen oder durch Umschlagen des Filzmaterials an den angrenzenden Flächen wird ebenfalls das Herausquellen des Absorbers in den Randgebieten verhindert.

Die Randversiegelung kann optional auch während des Schneidevorgangs beispielsweise durch Heißstanzen oder durch Schneiden mit einem heißen Werkzeug und Verschmelzen der Seitenränder erfolgen. Ebenso kann beim Lasern ein Verschmelzen der Seitenränder und somit eine Randversiegelung erfolgen. Weitere Verfahren zur Randversiegelung sind das Überspritzen der offenen Ränder, ein Steppen oder Filzen der Ränder, ein Verpressen der Ränder oder ein Umschlagen der überlappenden Flächen und Fixierung dieser Flächen durch eines der genannten Verfahren. Die Randversiegelung kann ebenso durch Überspritzen oder Hochfrequenz-Verschweißen erfolgen.

### Behandlung des Filzmaterials

Das erfindungsgemäße Filzmaterial weist in einer weiteren Ausführungsform behandelte Oberflächen, beispielsweise als Deckschicht oder behandelte Oberfläche, auf, wobei optional eine oder beide äußere Oberflächen der Filzschichten behandelt sind. Die Oberflächenbehandlung dient der Steuerung der Eigenschaften Wasserdurchlässigkeit, Luftdurchlässigkeit, Flüssigkeitsdurchlässigkeit, Staubdurchlässigkeit und/oder Regelung der Auf- und Abgabegeschwindigkeit des Wassers bzw. Wasserdampfs. Durch die Behandlung der Oberfläche können zudem die weiteren Eigenschaften des Filzmaterials, wie die Farbe und Materialeigenschaften wie Brandhemmung und Optik beeinflusst werden. Beispielsweise wird bei Bestrahlung eines schwarz eingefärbten Filzmaterials mit Sonnenlicht mehr Wärme vom Material aufgenommen, so dass die im Absorber gebundene Flüssigkeit aufgrund der höheren Wärme schneller verdunstet. Ebenso können beispielsweise Filterpigmente zugesetzt werden, die IR-Strahlen filtern, was ebenfalls zu einer veränderten Abgabegeschwindigkeit führt. Die Oberflächenbehandlung ermöglicht weiter durch Einpressen von Strukturen oder Abtragen von Material den Grad der Flexibilität zu bestimmen und/oder eine Vorgabe für die Richtung der Flexibilität zu treffen. Die Oberfläche kann weiterhin, wie bereits im Vorhergehenden beschrieben, mit Duft- oder Wirkstoffen, wie z.B. UV-Filtern,behandelt sein. Zudem ist auch eine biozide Ausrüstung der Oberfläche zum Schutz von Schädlingsbefall oder zum Schutz von Keim- oder Schimmelbefall möglich. Auch eine Ausrüstung der Oberfläche um einen Lotusblüteneffekt, d.h. eine Abweisung von Schmutz oder Öl, zu erreichen, ist möglich.

Die Oberflächenbehandlung erfolgt bevorzugt durch das Auftragen von Folien oder Schichten aus Metall, Kunststoff, Keramik/Polymergemische, gewebten Textilien (woven) oder nicht gewebten Textilien (non-woven), Leder oder luftdurchlässigen üblichen Flächenmaterialien oder durch Bedampfen mit Metall oder Ionisieren.. Zu Erhöhung der Luftdurchlässigkeit können diese Materialien perforiert sein. Bei Verwendung eines Materials mit Perforationen bzw. Vertiefungen werden diese vor oder nach dem Verbinden mit der Filzschicht in die Deckschicht eingebracht. Bevorzugt werden die Perforationen oder Vertiefungen erzeugt, indem die partiell die Deckschicht und ggf. Teile der Filzschicht entfernt oder verschoben werden. Dieses kann beispielsweise durch Ausbrennen, Lasern, Sandstrahlen, Ätzen, Bohren, Nadeln, Stechen oder stellenweise Auflockern der Fasern erfolgen.

Die Deckschicht kann beispielsweise durch Bedampfen z.B. mit einem Metall wie Silber, durch Auftragen von aushärtenden, trocknenden wirkstoff- oder farbstoffhaltigen Flüssigkeiten oder durch Tränken oder Beschlagen mit einer zum Zeitpunkt der Verarbeitung der flüssigen Substanz erfolgen Beim Tränken bzw. Beschlagen des Materials durchdringt dieses je nach Anwendungsbereich und Material die Filzschicht ganz oder nur in Teilbereichen. In einer weiteren Variante wird die Deckschicht auf die offenen Schnittkanten erstreckt. so dass die Deckschicht gleichzeitig die Randversiegelung bildet und in einem Arbeitsgang erfolgen kann.

In einer weiteren Ausführungsform ist das Filzmaterial so ausgeführt, dass sie zumindest in Teilbereichen versteift bzw. geformt ist. Die Versteifung des erfindungsgemäßen Filzmaterials kann durch Hitze und/oder Druck erreicht werden, wobei bestimmte Faseranteile der Filzschichten eine thermische Verbindung eingehen. Eine Versteifung des Filzmaterials kann alternativ durch Tränken oder Beschlagen mit einer zum Zeitpunkt der Verarbeitung flüssigen Substanz erfolgen, welche das Filzmaterial in Teilbereichen durchdringt. Durch das teilweise Tränken mit den aushärtenden Materialien werden gezielt Teilbereiche mit eingestellter Flexibilität geschaffen, so dass eine gezielte Weiterverarbeitung des erfindungsgemäßen Filzmaterials zu Formteilen möglich ist. In einer weiteren Alternative werden die Versteifungen als separates Element, beispielsweise in Form von Kunststoff- oder Metallformteilen eingebracht. Diese können beim Filzvorgang in das Filzmaterial integriert werden oder nachträglich eingebracht werden.

In einer weiteren Ausführungsform weist das erfindungsgemäße Filzmaterial Sperrelemente auf, die horizontal oder vertikal, bevorzugt vertikal/quer, zur Filzoberfläche, beispielsweise parallel zu den Verbindungsfasern angeordnet sind. Die Sperrelemente tragen ebenfalls zur Begrenzung der Ausdehnung des Absorbers bei. Die Sperrelemente werden beispielsweise durch Durchspritzung des Filzmaterials mit einem zum Zeitpunkt der Verarbeitung flüssigen Material, wie Kunststoff erhalten. Geeignete Sperrelemente liegen beispielsweise in Streifenform, Netzform oder Gitterform vor. Die Sperrelemente werden auch zur Stabilisierung des Filzmaterials bei Anwendung im Bereich mit hoher mechanischer Belastung eingesetzt. Weiter ist die Verwendung von Sperrelementen geeignet, wenn das erfindungsgemäße Filzmaterial zu Einsätzen oder Bauteilen verarbeitet wird, da die Sperrelemente dann als Randversiegelung genutzt werden können oder die Form des Bauteils aufweisen können. Die Sperrelemente erlauben es zudem, einzelne Bereiche abzugrenzen, zwecks Unterbindung der flächigen Ausbreitung von Feuchtigkeit oder Nässe. Diese können so auf einzelne Bereiche des Filzmaterials begrenzt werden, so dass nasse/feuchte und trockene Bereiche gebildet werden.

In einer weiteren Alternative werden zur Versteifung des erfindungsgemäßen Filzmaterials dauerhaft verformbare Fasern, Materialien oder schmelzbare Fasern eingesetzt, die eine Verformung und Verfestigung des Filzmaterials durch thermisches Pressen oder Aushärten durch Wärme oder UV-Bestrahlung ermöglichen. Die Einbringung einer Versteifung in das erfindungsgemäße Filzmaterial ist insbesondere für die technischen Anwendungsbereiche, wie die medizinischen Anwendungsbereiche, des erfindungsgemäßen Filzmaterials relevant. So ist durch den Einsatz von Versteifungen, wie anpassbare immobilisierende medizinische Schienen, medizinische Verbände, orthopädische Einlagen oder technische Dichtungsmaterialien, eine Anpassung vor Ort möglich. Dieses gilt ebenso für den technischen Anwendungsbereich, in dem das Material klimaregulierend eingesetzt wird, z.B. zur Kanalsanierung oder für Verpackungsmaterialien, wie z.B. Container.

Die Oberfläche des erfindungsgemäßen Filzmaterials ist in einer Variante mit Abstützhaltem ausgerichtet, die der Vermeidung einer Feuchtigkeitsbrücke oder der Vermeidung eines flächigen Kontaktes z.B. bei starker Hitze oder Kälte entwickelnden Flächen oder Isolation gegen Elektrizität dienen.

Um die Bildung einer Feuchtigkeitsbrücke zum umliegenden Material bzw. zum Träger der entsprechenden Gegenstände zu verhindern, wird bevorzugt auf der Körper zugewandten Seite ein nicht wasserleitendes Material eingesetzt oder das Material entsprechend ausgerüstet. Die Bildung einer Feuchtigkeitsbrücke wird ggf. zusätzlich durch die Kunststoffanteile, wie Trägerschichten, Schutzgitter und Distanzflächen verhindert. Das Verhindern eines direkten Körperkontaktes mit entsprechenden Flächen hat nicht nur den Vorteil, dass eine Luftschicht ausgebildet wird, sondern zudem auch, das Kälte bzw. Temperaturunterschiede von außen nicht direkt durch das feuchte Material des Absorbers oder andere Bestandteile des Belüftungseinsatzes an den Träger weitergeleitet werden. Die körperzugewandten Strukturen haben den Vorteil, dass zum einen ein Luftstrom an der Körperseite zirkulieren kann und zum anderen beim starken Schwitzen der taktile Kontakt durch Wassertropfen, wie z.B. Schweiß, das erfindungsgemäße Filzmaterial nicht dazu veranlasst, sich durch eine Quellung vorzeitig zu verschließen und so die Belüftung zu unterbrechen. Für die dem Körper zugewandten Materialien werden beispielsweise hautfreundliche, aber nicht wasserleitende Materialien oder hautfreundliche, wasserleitende Materialien eingesetzt. Solche Materialien sind beispielsweise Mikrofasern bzw. Membranen, welche in Teilbereichen, in denen sich der Absorber befindet, über luftdurchlässige Öffnungen verfügen und so die luftdurchlässige Schicht bilden. Durch den Einsatz solcher Materialien kann der Vorteil der Wasserdampfdurchlässigkeit dieser Materialien mit der verbesserten Luftdurchlässigkeit des erfindungsgemäßen Filzmaterials verbunden werden.

In einer weiteren Ausführungsform enthält das erfindungsgemäße Filzmaterial in mindestens einer der Filzschichten und/oder der absorberhaltigen Schicht oder als zusätzliche Schicht zusätzliche Materialien, die geeignet sind, bestimmte Stoffe gezielt aufzunehmen oder unter bestimmten Umweltbedingungen abzugeben. Die Materialien sind beispielsweise geeignet, um Wirkstoffe, Duftstoffe, Reinigungsstoffe, Schleifmittel, Mikroorganismen, Medikamente, Pestizide, Fungizide, Enzym- oder Farbstoffe aufzunehmen oder freizusetzen. Im Falle der Freisetzung werden bevorzugt Materialien eingesetzt, die eine Freisetzung der Funktionsstoffe abhängig von folgenden Parametern erlauben:
- pH-Wert,
- Temperatur,
- Salzgehalt,
- Art der Flüssigkeit oder des Gases,
- chemische Substanzen,
- Enzyme,
- Druck,
- mechanischen Einflüssen,
- chemischen Einflüssen,
- Trocknungsdauer,
- Benetzungsdauer und/oder
- Häufigkeit,
- Strom/Spannung.

Beispielsweise ist das erfindungsgemäße Filzmaterial mit einem Material versehen, das als Indikator wirkt, wobei zunächst ein Wasserkontakt oder ggf. Wasserdampfkontakt der Fläche notwendig ist, um den Indikator zu aktivieren. Die Färbung des Indikators gibt dabei beispielsweise den Sättigungsgrad oder die noch verbleibende Funktionsdauer nach dem Kontakt mit schädlichen Substanzen an. Es ist auch denkbar, mehrere solcher Reaktionen von Funktionsstoffen miteinander zu koppeln oder in Reihe zu schalten.

Das Filzmaterial ist ggf. so ausgestaltet, dass es waschbar oder durch chemische Reinigung zu säubern ist. Durch die mechanische Festigkeit lässt sich Staub oder Schmutz auch mechanisch vom Filzmaterial entfernen, z.B. durch Ausschlagen, Abblasen oder Absaugen.

Bevorzugt ist das erfindungsgemäße Filzmaterial maschinenwaschbar. Das maschinenwaschbare Filzmaterial ist dabei üblicherweise so ausgeführt, dass die Kanten des Filzmaterials verschlossen sind, um ein teilweises Austreten des Absorbers zu verhindern. Bei nicht verschlossenen Kanten des erfindungsgemäßen Filzmaterials tritt bei einer Waschmaschinenreinigung der Absorber in den seitlichen Teilbereichen in sehr geringen Mengen aus. Der wesentliche Anteil der Absorberpolymerpartikel wird durch die senkrecht laufenden Filzfasern zwischen den Filzschichten im Material gehalten. Ein flächiges Austreten des Absorberpolymers wurde daher auch im nassen Zustand bzw. bei der Wäsche nicht beobachtet. Die Waschbarkeit des erfindungsgemäßen Filzmaterials stellt einen wesentlichen Vorteil gegenüber den aus dem Stand der Technik bekannten absorberhaltigen Materialien dar, die alle nicht waschbar, insbesondere nicht maschinenwaschbar sind.

Weiter wurde beobachtet, dass bei der Maschinenwäsche bzw. der Wäsche eine Quellung des Absorberpolymers auftritt, die zumindest teilweise zu einer Auflockerung des Filzes führt, da durch die Quellkräfte des Absorberpolymers die Fasern abhängig von der Faserbeschaffenheit und dem Grad des Nadelfilzens teilweise verschoben werden. Zudem bewirkt die Maschinenwäsche bei einigen Ausführungsformen des erfindungsgemäßen Filzmaterial eine zusätzlich Verfilzung besonders der außen liegenden Schichten, wodurch eine weitere stärkere Einbindung des Absorberpolymers zwischen den Schichten erfolgt und das Absorberpolymer am Austreten gehindert wird.

### Herstellung des Filzmaterials

Erfindungsgemäß erfolgt die Herstellung eines Filzmaterials durch Nadelfilzen, bei dem das Fasermaterial mechanisch mit zahlreichen Nadeln mit Widerhaken durchdrungen wird. Durch das wiederholte Einstechen werden die Fasern der verschiedenen Faserschichten miteinander verschlungen, so dass ein Filz- oder Nadelvliesstoff entsteht. Durch das Nadelfilzen wird das Fasermaterial verdichtet, so dass ein einheitliches textiles Flächegebilde entsteht.

Alternativ wird das Filzmaterial weiter verdichtet. Die weitere Verdichtung wird durch Hitze und/oder Druck erreicht oder durch Tränken des Filzmaterials mit zum Zeitpunkt der Verarbeitung flüssigen Substanzen. Das erfindungsgemäße Verfahren zur Herstellung des Filzmaterials wird so durchgeführt, dass die Fasern der einen Filzschiebt zumindest die absorberhaltige Schicht, bevorzugt die weitere Filzschicht, durchdringen. Dabei durchdringen primär die Fasern der ersten Schicht die zweite Schicht. Es ist sowohl möglich, dass das reiche Durchdrihgungsverhättnis aller Faserschichten untereinander erreicht wird, als auch dass beim Herstellungsverfahren unterschiedliche Durchdringungsverhälthisse für die verschiedenen Faserschichten erreicht werden.

Die Schichten, aus denen der verbindende Faseranteil stammt, werden deshalb ggf. in ihrer Materialbeschaffenheit und der Menge der Fasern an das gewünschte Material angepasst Erfindungsgemäß werden sowohl Materialien hergestellt, bei dem die Durchdringung von Verbindungsfasern der ersten Filzschicht durch die zweite Filzschicht erfolgt, bei dem die Durchdringung von der zweiten Filzschicht durch die erste Filzschicht erfolgt oder bei denen die Durchdringung der ersten und der zweiten Filzschicht durch das Trägermaterial der absorberhaltigen Schicht bzw. die absorberhaltige Schicht erfolgt.

Ein Superabsorber beinhaltendes Filzmaterial kann durch Kaltfilzen als Meterware hergesteiftwerden und ggf. durch einen weiterverarbeitenden Betrieb konfektioniert, mit Formteilen verbunden oder anderweitig veredelt (Lotusblüteneffekt, Silberionen) werden.

Die Herstellung des erfindungsgemäßen Filzmaterials erfolgt in einer Anwendung so, dass der Filz in einer an die Größe der Endanwendung angepassten Größe gefilzt wird. Dieses ist möglich, da das erfindungsgemäße Filzmaterial erfindungsgemäß auch durch kleihflächiges Filzen hergestellt werden kann.

Erfindungsgemäß wird optional bei der Herstellung des Filzmaterials Funktionsmaterial an beliebigen Stellen der Filzschicht eingefilzt. Beispielsweise ist es möglich, das Funktionsmaterial nur an den Seitenrändern einzufilzen.

Als Funktionsmaterial enthält das erfindungsgemäße Filzmaterial bespielsweise Formgedächtnismateriatien, die durch Temperaturunterschiede oder andere Auslöser wie UV-Strahlung, Hitze, Kälte, Dampf, Strom oder Spannung aktiviert werden. Diese Funktionsmaterialien könnten Formgedächtnisreaktionen verursachen und hierdurch die Beschaffenheit des Filzes einstellen und hierdurch eine andere mechanische Verfestigung oder Verbindung mit anderen Fasern eingehen, als nicht aktivierte Formgedächtnismaterialien. Die Formgedächtnismaterialien sind in einer Ausführung Teil der Oberflächenbehandlung und unterstützen z.B. zusätzlich Verschluss oder Öffnungsmechanismen an der Oberfläche. Ebenso werden die Formgedächtnismaterialien z.B. als Indikator für die Benetzung bzw. den Beladungszustand des Absorbers genutzt.

Bei einer Ausführungsform des erfindungsgemäßen Verfahrens wird das Filzmaterial so hergestellt, dass es Soll-Knickstellen oder Soll-Bruchstellen enthält. Die Soll-Knickstellen bzw. Soll-Bruchstellen können durch verschiedene Verfahren erzeigt werden, wie z.B. Schneiden, Anschneiden, Einbringen von Substanzen, Perforieren, Lochen, Stechen, Abtragen, Pressen, Anschmelzen, Anstanzen, Durchstanzen, Lasern, chemisch abtragen oder verdichten oder Behandlungen mit entsprechenden Laminaten oder Überzügen.

In einer Ausführungsform des erfindungsgemäßen Verfahrens erfolgt die Herstellung des Filzmaterials durch Nadelfilzen und zusätzliches Nassfilzen. Hierdurch wird die Oberfläche des Filzmaterials zusätzlich verdichtet und es kann durch das Nassfilzen auch beispielsweise in Verbindung mit Laugen oder verdünnten Säuren eine Verformung des Filzes erreicht werden. Das Verfahren wird dabei in einer Variante so durchgeführt, dass Teilbereiche durch Nadelfilzen und andere Teilbereiche durch Nassfilzen verfilzt werden. Die beiden Filztechniken werden bei dieser Ausführungsform je nach gewünschtem Filzmaterial beliebig miteinander kombiniert.

Bei der Herstellung des erfindungsgemäßen Filzmaterials lassen sich die Eigenschaften des entstehenden Filzes durch die Stärke, Dauer, Anordnung, Summe, Dichte und Funktionalität der Nadeln beeinflussen. So führt beispielweise eine unterschiedliche Länge der Nadeln zu einer unterschiedlichen Durchdringung und somit zu einem unterschiedlich starken Faserverbund zwischen den Schichten.

Optional sind auf einem Nagelbrett der Filzmaschine Nadeln mit unterschiedlichen Widerhaken, unterschiedlichen Durchmessern, unterschiedlichen Längen und/oder unterschiedlichen Formen angeordnet.

Optional werden beim erfindungsgemäßen Verfahren Filzhilfsmittel verwendet, welche die mechanische Festigung des Filzes beeinflussen. Die üblichen Filzhilfsmittel werden dabei entweder über die gesamte Filzbreite oder nur in Teilbereichen eingesetzt.

Bei einer weiteren Ausführungsform des erfindungsgemäßen Verfahrens werden als Nadeln zumindest teilweise Hohlnadeln eingesetzt, so dass durch die Hohlnadeln gezielt Substanzen in das Material eingefilzt werden können. Bevorzugt werden hier Substanzen eingebracht, die zu einer gezielten Beeinflussung der Filzeigenschaften führen. So können Gase beispielsweise gezielt die Fasern aufrauen. Ebenso ist es beispielsweise möglich, auf diesem Wege Materialien einzubringen, die Formgedächnisreaktionen verursachen bei Temperaturunterschieden oder anderen auslösenden Einflussgrößen, wie Hitze, Kälte, Dampf oder UV-Bestrahlung. Hierdurch lässt sich ebenfalls die Beschaffenheit des Filzmaterials einstellen, da Bereiche, in denen aktivierte Funktionsfasern eingebracht sind, eine andere mechanische Verfestigung und einen anderen Faserverbund aufweisen, als Bereiche mit nicht aktivierten Funktionsmaterialien.

Während des erfindungsgemäßen Filzvorgangs werden bei einer Ausführung die Filznadeln erhitzt oder gekühlt. Ein Erhitzen der Filznadeln ist besonders dann bevorzugt, wenn Teile des zu filzenden oder überfilzenden Materials aus Schmelzfasern bestehen. Durch die von den Filznadein abgegebene Wärme erfolgt dann eine bessere Verbindung der Fasern, mit den nicht erwärmten Fasern. Alternativ wird das zu filzende oder überfilzende Material erhitzt und durch gekühlte Nadeln in Teilbereichen abgekühlt, so dass hier ebenfalls unterschiedliche Faserverbundstärken und Materialeigenschaften erreicht werden können.

In einem optionalen weiteren Verfahrensschritt lässt sich die Verdichtung des Faserfloors während des Filzvorgangs zusätzlich zum Nadelfilzen durch Hitze oder Druck nach dem Filzen verstärken.

Bei Filzmaterialien, die zusätzlich eine Abdeckschicht oder Laminatschicht aufweisen, wird in einer Variante die Abdeckschicht bzw. das Laminat durch Anfilzen an einer der Filzschichten fixiert. In einem darauf folgenden Verarbeitungsschritt erfolgt dann das eigentliche Filzen. Der Verarbeitungsschritt des Anfilzens des Laminats und das anschließende vollständige Filzen kann in einem unmittelbar nacheinander ablaufenden Verarbeitungsschritt erfolgen. Ebenso ist es möglich, einen bereits begonnenen Filzvorgang zu unterbrechen und dann das Laminat oder die Abdeckschicht aufzulegen und den Filzvorgang mit dieser Schicht fortzusetzen.

Bei der Herstellung des erfindungsgemäßen Filzmaterials werden in einer Ausführungsform die Schichten unmittelbar vor der mechanischen Verfestigung erwärmt oder gekühlt, um so eine bessere Verbindung oder Verbindungsbereiche mit unterschiedlichen Eigenschaften zu erreichen. Wenn die verschiedenen Schichten als Bahnen unmittelbar vor dem Filzen zusammengeführt werden, ist es auch denkbar, einzelne Bahnen mit unterschiedlicher Temperatur zusammenzuführen oder alle Bahnen auf die gleiche Temperatur zu erhitzen oder abzukühlen. Zudem ist es auch möglich, während des Filzvorgangs unterschiedliche Temperaturbereiche innerhalb des Filzmaterials einzustellen, da die Temperaturen abhängig von der Frequenz des Nadelfilzens unter Form und Beschaffenheit der Nadeln ist.

Bei einer weiteren Ausführungsform des erfindungsgemäßen Verfahrens wird die absorberhaltige Schicht mit einem Vorprodukt des Absorberpolymers eingebracht. Die eigentliche Polymerisation zum Absorberpolymer läuft dann während des Filzvorgangs oder nachdem der Filzvorgang beendet wurde, ab. Wird das Verfahren mit Hilfe von Hohlnadeln ausgeführt, so lässt sich das Absorberpolymer oder ein Vorprodukte des Absorberpolymers durch die Hohlnadeln während des Filzvorgangs in das Material einbringen.

In einer weiteren Ausführungsform wird das erfindungsgemäße Verfahren so durchgeführt, dass gequollene oder angequollene Absorberpolymere in der absorberhaltigen Schicht eingesetzt werden und diese überfilzt werden. Nach der Trocknung der absorberhaltigen Schicht entstehen so definierte Zwischenräume im erfindungsgemäßen Filzmaterial. Auf diese Weise lässt sich beispielsweise die Beladungsmenge, die ein erfindungsgemäßes Filzmaterial maximal aufnehmen kann, gezielt einstellen. Wenn beispielsweise das erfindungsgemäße Filzmaterial 2 l/m² Flüssigkeit aufnehmen soll, wird diese Menge der absorberhaltigen Schicht zugegeben und diese dann verfilzt. Bei der Verwendung von gering dehnbaren Filzfasern ist somit die maximale Aufnahmemenge des Filzmaterials vordefiniert.

Es hat sich gezeigt, dass das vorgequollene Absorbermaterial weicher ist als das noch nicht gequollene und nicht benetzte Absorberpolymer. Entsprechend ist das vorgequollene oder gequollene Absorbermaterial leichter zu verarbeiten. Dieses hängt vermutlich damit zusammen, dass viele Materialien im nassen, weichen Zustand besser federn und eine höhere Elastizität haben als trockenes hartes Material. Hierdurch wird der Filzvorgang erleichtert.

In einer weiteren Ausführungsform des erfindungsgemäßen Verfahrens werden die Verbindungsfasern des Filzmaterials durch eine nicht faserhaltige Schicht getrieben. Eine solche Schicht ist beispielsweise ein Laminat oder eine Kunststoffschicht. Zur besseren Verzahnung der Schichten miteinander wird die durch die Nichtfaserschicht getriebene Faser nach dem Filzvorgang umgeknickt, beispielsweise durch Walzen, Rollen oder Pressen. Wenn es sich bei den Fasern um Schmelzfasern handelt, können diese durch Hitze oder Druck aufgepresst werden und so auf der nichtfaserhaltigen Schicht fixiert werden. Grundsätzlich ist es auch möglich, die nichtfaserhaltige Schicht so auszugestalten, dass diese haftend gestaltet ist und die Verbindungfasern beim Umknicken mit dieser Schicht verkleben bzw. auf dieser Schicht fixiert werden. Die nichtfaserhaltige Schicht kann hierzu beispielsweise thermoplastisch ausgebildet sein oder mit einer Klebstoffschicht versehen sein.

Erfindungsgemäß wird in einer Variante über das Verfahren zur Herstellung des Filzmaterials auch ein dreidimensionaler Formkörper hergestellt werden. Dieser dreidimensionale Formkörper wird durch gezieltes Filzen der jeweiligen Formen, durch Schleifen, Schneiden oder Abtragen aus einem entsprechenden Filzgrundkörper erzeugt. Alternativ lässt sich das erfindungsgemäße Filzmaterial auch als Schlauch herstellen.

### Bauteil aus Filzmaterial

Gegenstand der Erfindung ist weiterhin ein Bauteil aus dem erfindungsgemäßen Filzmaterial, wie im Vorhergehenden beschrieben.

Das Bauteil wird bevorzugt durch Ausschneiden oder Ausstanzen aus dem Filzmaterial in Meter- oder Plattenwaren, wie im Vorhergehenden beschrieben, erhalten. Das Bauteil wird dann ggf. mit einem der vorgenannten Verfahren randversiegelt, um ein Austreten des Absorbers an den offenen Schnittkanten zu vermeiden. In einer weiteren Ausführungsform weist das Bauteil an seinen Rändern Verbindungselemente auf, die ein Einpassen des Bauteils in einem Bekleidungsstück, Schuhwerk oder einem anderen Anwendungsgegenstand ermöglichen. Die Verbindungselemente können beispielsweise durch Überspritzen mit einem Kunststoff, Schraubverbindungen, Klebeverbindungen, Schweißverbindungen, Klemmverbindungen oder Steckverbindungen erzeugt werden.

Bei der Ausführungsform der Bauteile mit Verbindungselement sind diese bevorzugt als Verlängerung der Filzschicht ausgebildet. Die Verbindungselemente dienen zur Befestigung des Bauteils an dem zu belüftenden, zu entfeuchtenden oder zu dichtenden Gegenstand oder Material. Die Verbindung an den Verbindungselementen kann beispielsweise durch Magnetverbindung, Klickverbindung, Steckverbindung, Schiebeverbindung, Stülpverbindung, Klebverbindung, Falzverbindung, thermoplastisches Verschweißen oder Nähte hergestellt werden. Mit Hilfe der Verbindungselemente wird das erfindungsgemäße Bauteil so über einer Öffnung in.der zu belüftenden Textil oder dem zu belüftenden Gegenstand angeordnet, dass er diese Öffnung vollständig bedeckt. In einer Ausführungsform sind die Verbindungselemente dabei so ausgestaltet, dass das erfindungsgemäße Bauteil austauschbar ist. Das Bauteil kann dabei als Einmalartikel konstruiert sein, der nach einmaligem Gebrauch ausgetauscht wird. Das Bauteil ist weniger bevorzugt als nicht reversibel sich abdichtende Fläche verarbeitet.

In einer Ausführungsform sind die Verbindungselemente so ausgestaltet, dass ein Austauschen des Bauteils durch ein neues Bauteil möglich ist. Ein solcher Austausch kann auch dazu dienen, um ein Bauteil mit anderen Eigenschaften, beispielsweise einen Belüftungseirisatz der zusätzliche Funktionen aufweist an dem zu belüftenden Objekt einzusetzen. Diese Ausführungsform wird beispielsweise insbesondere bei Schutzhelmen oder Elektroartikeln eingesetzt.

In einer weiteren Ausführungsform sind die Verbindungselemente mit Strukturen oder Aus- bzw. Einwölbungen versehen, die ein, ggf. passgenaues, Befestigen des Bauteils am zu belüftenden Objekt erlauben. Die Verbindungselemente sind beispielsweise zusätzliche Bauteile und die Verbindung kann beispielsweise durch Umspritzen mit Kunststoff, kleben, schrauben, nieten, Steckverbindungen oder nähen erfolgen. Die Herstellung der Verbindungselemente erfolgt in unterschiedlichen Stärken, je nach Anwendungsbereich. Die Flächen der Elemente sind bevorzugt dünn und laufen nach außen hin auf 0 zusammen. Bevorzugt können die Verbindungselemente bereits während der Spritzgussherstellung texturiert werden oder nachträglich durch mechanisches Aufrauen, Ätzen, Bestrahlen mit UV-Licht oder Behandlung mit Gasen, wie Ozon textuiert werden. Eine entsprechend texturierte Oberfläche der Verbindungselemente erleichtert die Befestigung am zu belüftenden Objekt, wenn Verbindungsweisen wie kleben oder verschweißen oder ähnliches gewählt werden.

Das erfindungsgemäße Bauteil kann wie das erfindungsgemäße Filzmaterial für viele verschiedene Anwendungsbereiche, bei denen eine Luftzufuhr, Belüftung oder Entfeuchtung einerseits und ein Schutz vor Wasser oder anderen Flüssigkeiten andererseits gewünscht ist, eingesetzt werden.

In einer weiteren Ausführungsform ist das Bauteil ohne Randversiegelung ausgestaltet, aber optional mit einem Rahmen, der die Seitenränder des Filzformteils bündig umschließt. Das Bauteil wird dann so in den Rahmen bzw. zu belüftende Objekt eingesetzt, dass der Rahmen oder die Ränder des zu belüftenden Objekts ein Austreten des Absorbers verhindern und diesen in seiner Ausdehnung mitbegrenzen. Das erfindungsgemäße Bauteil ist dann so konfektioniert, dass es in das Endprodukt eingepasst und dort fixiert werden kann.

In einer weiteren Ausführungsform ist das Bauteil so ausgestaltet, dass das erfindungsgemäße Filzmaterial teilweise mit Kunststoff überspritzt, unterspritzt oder hiermit versehen ist. Das erfindungsgemäße Filzmaterial und der Kunststoff liegen dabei entweder als Materialverbund vor oder als voneinander abtrennbare Bestandteile. Bezogen auf den mit dem Filzmaterial versehenen Gegenstand ist das erfindungsgemäße Filzmaterial dabei entweder vollständig oder teilweise außenliegend oder innenliegend angeordnet. Beispielsweise bei der Verwendung für Griffe von Werkzeugen wird der Kunststoffgriff innen am Werkzeug liegen und von außen mit dem Filzmaterial umschlossen. Bei einer anderen Gestaltung ist beispielsweise der textile Teil als Innenschuh eines Gummistiefels ausgebildet und mit einem Kunststoffmaterial zur Bildung des Gummistiefels überspritzt. Eine vergleichbare Kombination von Kunststoffmaterial und Filzmaterial ist auch bei aufgespritzten oder gegossenen Knieschützern, Schutzhelmen oder Schuhsohlen möglich.

### Verwendung des erfindungsgemäß Filzmaterials und des erfindungsgemäßen Bauteils

Das erfindungsgemäße Filzmaterial und die Bauteile aus dem erfindungsgemäßen Filzmaterials werden überall dort eingesetzt, wo eine Klimaregulierung, wie Belüftung oder Entfeuchtung, d.h. eine Zufuhr von Luft gewünscht ist und gleichzeitig eine Berührung mit Wasser oder Flüssigkeiten vermieden oder eine gezielte Zuführung bzw. Abgabe von Wasser erreicht werden soll. Entsprechend gibt es eine Vielzahl von Anwendungsmöglichkeiten für das erfindungsgemäße Filzmaterial sowohl für textile als auch nicht textile Gegenstände. Abhängig vom jeweiligen Gebiet dient das erfindungsgemäße Filzmaterial bzw. das Bauteil zur Klimaregulierung in Form einer Belüftung, wobei die Sperrfunktion, die zu einem Selbstschließen des Materials führt, vor Wasser oder Flüssigkeiten schützt. Aufgrund der mechanischen Festigkeit kann das Material dabei wesentlich höheren Belastungen ausgesetzt werden, als übliche absorberhaltige Materialien. Auch zur Abdichtung von Flächen oder zur Entfeuchtung, d.h. zum gezielten Abtransport von Wasser oder Wasserdampf wird das erfindungsgemäße Material vorteilhaft eingesetzt. Das erfindungsgemäße Material wird somit insbesondere zu Klimaregulierung durch Belüftung, zur Klimaregulierung durch Entfeuchtung oder Befeuchtung und zum selbsttätigen Abdichten von textilen und nicht-textilen Gegenständen eingesetzt.

Eine Verwendungsmöglichkeit des erfindungsgemäßen Filzes besteht im Einsatz als Schuhobermaterial, das bedingt durch die Eigenschaften des Filzes luftdurchlässig ist und durch die Ausrüstung mit der absorberhaltigen Schicht auch wasserdicht. Zur Verwendung als Schuhmaterial wird das erfindungsgemäße Filzmaterial beispielsweise in Form gestanzt und ggf. weiter bearbeitet zur Randversiegelung der offenen Schnittkanten oder dem Aufbringen einer Oberflächenbehandlung. Alternativ wird das Filzmaferial bzw. die Bauteile angespritzt oder teilweise Überspritzt, z.B. zur Herstellung eines Gummistiefels. Ein Schuhobermaterial aus dem erfindungsgemäßen Filzmaterial oder mit den erfindungsgemäßen Bauteilen hat den Vorteil, dass er aufgrund der Luftdurchlässigkeit auch feuchtigkeitsregutierend wirkt, da Wasserdampf aus dem Schuhinneren heraus transportiert wird.

Eine weitere Verwendungsmöglichkeit des erfindungsgemäßen Filzes besteht im Einsatz in der Schuhsohle oder für Schuhinnensohlen. Als Bauteil in der Schuhsohle ermöglicht der erfindungsgemäße Filz sowohl eine Belüftung des Fußes als auch einen Abtransport von Feuchtigkeit, wie Körperschweiß, aus dem Schuh. Bei Berührung mit Nässe, beispielsweise durch eine Pfütze oder Regen, verschließt sich die Schuhsohle selbsttätig. Bei einer weiteren Verwendung in der Schuhsohle ist die Innensohle oder Einlegesohle so mit Absorbern verstehen, dass sich durch eine Quellung des Absorbers die Stoßabsorbtion des erfindungsgemäßen Filzes verändert. Die Sohle bzw. der Schuh weist somit bei Nässe andere Dämpfungseigenschaften als bei Trockenheit auf.

Eine weitere Verwendung des erfindungsgemäßen Filzmaterials stellt die Belüftung von Kleidurigsstücken dar. Hierbei wird das erfindungsgemäße Filzmaterial beispielsweise auch als Futtermaterial oder als Teil des Oberflächenmaterials der Bekleidung eingesetzt. Optional können auch Bauteile aus dem erfindungsgemäßen Filzmaterial als Belüftungseinsatz in die Kleidung eingebracht sein. Es ist so eine Luftzufuhr und Belüftung während des Tragens der Bekleidungsstücke, wie z.B. Jacken, Hosen, Mützen oder Westen möglich. Hierdurch wird der Tragekomfort erhöht, da Feuchtigkeit, die sich im Kleidungsstück bildet, nach außen transportiert wird. Gleichzeitig besteht jedoch nicht das Risiko, dass Wasser oder Flüssigkeit in das Innere des Bekleidungsstückes eindringt, da sich in diesen Fällen das erfindungsgemäße Filzmaterial bzw. die Bauteile mit Wasserkontakt sofort verschließen würden.

In einer Ausführung ist das erfindungsgemäße Filzmaterial bzw. Bauteil aus dem erfindungsgemäßen Filzmaterial dabei als Bauteil/Funktionselement nicht sichtbar in die Kleidung eingearbeitet. So wird ein Funktionselement aus dem erfindungsgemäßen Filz beispielsweise mit einer Stofflage oder einer Lage eines anderen Funktionsmaterials, z.B. einer Klimamembran oder anderen wasser-, wasserdampfoffenen Materialien überdeckt.

Bei Verwendung des erfindungsgemäßen Filzmaterials bzw. der Bauteile zur Entfeuchtung von Bekleidung wird das Material beispielsweise als Saugpads im Achselbereich von Bekleidungsstücken, wie z.B. Jacken oder Sakkos, eingesetzt. Das erfindungsgemäße Material saugt die Feuchtigkeit auf und gibt diese durch Verdunstung nach und nach an die Umgebung ab. Hierdurch kann vermieden werden, dass sich das Kleidungsstück in den jeweiligen Bereichen unangenehm nass anfühlt. Durch die mechanische Festigkeit des erfindungsgemäßen Materials können die Einsätze dauerhaft im Bekleidungsstück verbleiben, was bei bisherigen absorberhaltigen Materialien nicht möglich war, da diese nicht waschbar sind und durch die hohe Volumenzunahme nicht "unsichtbar" in das Kleidungsstück oder das Futter des Kleidungsstücks integriert werden konnten.

Die Wasser- bzw. Feuchtigkeitsaufnahme des erfindungsgemäßen Filzmaterials bzw. der Bauteile ermöglicht auch die Verwendung als Entfeuchtungseinsatz in Bereichen, in denen sich Kondenswasser sammelt, wie z.B. Zelten, Verpackungen oder Gehäusen von elektronischen Geräten. In diesen Fällen nimmt der Entfeuchtungseinsatz das ggf. an den Wänden herunterrinnende und in den Einsatz rinnende Wasser auf und gibt diese nach und nach an die Umgebung ab. Hierdurch erfolgt eine Klimaregulierung in dem Zelt bzw. Behälter.

Eine weitere Verwendung sieht vor, dass das erfindungsgemäße Filzmaterial bzw. die Bauteile zur Klimaregulierung in Spezialverpackungen bzw. technischen Anwendungen eingesetzt werden können. Ist das Innere der Verpackung sehr trocken und sollte beispielsweise bei der Lagerung von verderblichen Gütern wie Obst ein bestimmtes Feuchtigkeitsmaß nicht unterschreiten, so kann durch das Filzmaterial bzw. dem Bauteil bei sehr trockener Luft Wasserdampf in das Verpackungsinnere transportiert oder an dieses abgegeben wird.

Eine weitere Verwendungsmöglichkeit besteht in verschiedenen Einsatzbereichen für Fußbodenbeläge. Zum einen ist es möglich, dass das erfindungsgemäße Filzmaterial in Teppichboden einzusetzen. Das Filzmaterial wird in diesem Fall als Träger oder Fixierungsschicht für die Fluorfasern des Teppichbodens verwendet. Die Fluorfasern ragen beispielsweise durch den Filz und haben einen Kontakt zur absorberhaltigen Schicht, so dass sie zum Transport von Wasser und Feuchtigkeit dienen. Ein entsprechend ausgerüsteter Teppichboden erlaubt die schnelle Aufnahme von Flüssigkeit, die gezielt in die absorberhaltige Schicht geleitet wird, so dass der Teppichflor schneller abtrocknet und keine Flüssigkeitslache auf dem Teppich steht. Aus der absorberhaltigen Schicht wird die Feuchtigkeit dann durch Verdampfen langsam und gleichmäßig abgegeben. Zum anderen eignet sich das erfindungsgemäße Filzmaterial auch als Zwischenschicht oder Trittschalldämmung für harte Bodenbeläge wie z.B. Platten, Parkett oder Laminat. Hierdurch können ebenfalls größere Flüssigkeitsmengen aufgenommen werden und beispielsweise ein Aufquellen von Laminat oder Parkett verringert oder vermieden werden. Ein Einsatz ist überall dort sinnvoll, wo der Bodenbelag als solcher, sei es der Teppichflor oder das harte Material, vor Feuchtigkeit geschützt werden und Feuchtigkeit gezielt abgeführt werden soll. Zudem hilft der Einsatz des erfindungsgemäßen Filzmaterials in Bodenbelägen das Raumklima zu verbessem.

Im Bausektor ist die Verwendung des erfindungsgemäßen Filzmaterials als Zwischenabdichtung z.B. zur Wand oder in Form von Filzfliesen sowohl im Boden-, Decken- als auch Wandbereich möglich. Weiterhin ist ein Einsatz als Dachziegel oder Bestandteil von Dachabdeckungen möglich, wie z.B. Dachdämmbahnen, Dachbegrünungen, Isolierschichten oder Dachbahnen.

Eine weitere Verwendungsmöglichkeit im Baubereich besteht als Belag in Form des Unterputzes oder als Wandbelag. Hierdurch wird die Wand effektiv vor aufsteigender Feuchtigkeit geschützt im Gegensatz zu anderen offenen oder kapillaren Materialien. Aufgrund der mechanischen Kontrollierbarkeit ist das erfindungsgemäße Filzmaterial besondern für die Anwendung auch als Wandbelag geeignet.

Die Wasseraufnahme und Wasserabgabe des erfindungsgemäßen Filzmaterials wird weiter bei der Verwendung für Bewässerungsleitungen oder Bewässerungselemente für Pflanzen eingesetzt. Die Bewässerungsleitungen, die in das Erdreich oder in ein Wuchssubstrat eingebracht werden, bestehen teilweise oder ganz aus dem erfindungsgemäßen Filzmaterial. Die Bewässerungsleitung aus dem erfindungsgemäßen Material gibt jeweils nur so viel Wasser aus dem Inneren an das Erdreich bzw. das Wuchssubstrat ab, wie von diesem aufgenommen werden kann. Teilbereiche der Bewässerungsleitungen bzw. Bewässerungselemente, in denen sich kein Wasser befindet, sind zudem offen für Luft, so dass hierdurch eine Belüftung des Erdreichs bzw. Wuchssubstrats erfolgt. Zudem besteht auch die Möglichkeit über die nicht mit Wasser gefüllten Bereiche gezielt wachstumsfördernde Medien oder Gase in der Erdreich einzubringen. Hierdurch ist eine Belüftung auch bei solchen Pflanzsystemen möglich, bei denen das Substrat bzw. Erdreich mit Folien bedeckt ist, um Wasser und Wärme zurückzuhalten. Es können durch Einsatz der Bewässerungsleitungen bzw. Bewässerungselemente aus dem erfindungsgemäßen Material sowohl Staunässe als auch Austrocknung des Erdreichs gezielt vermieden werden.

Bei einer weiteren Verwendung des erfindungsgemäßen Filzmaterials wird dieses als Kondenswassersammler eingesetzt. Beispielsweise können Bahnen aus dem erfindungsgemäßen Filzmaterial liegend oder vorzugsweise mehr oder weniger aufrecht bis zu senkrecht positioniert werden, um Nebel, Taufeuchte oder Luftfeuchtigkeit aufnehmen. Bevorzugt ist die Oberfläche des erfindungsgemäßen Filzmaterials in diesem Fall strukturiert, z.B. schuppig oder streifenförmig, mit bevorzugt nach oben gerichteten Öffnungen, um die Wasseraufnahme zu erhöhen. Durch eine Strukturierung der Oberfläche kann das Wasser besser in die absorberhaltige Schicht des erfindungsgemäßen Filzmaterials geleitet werden. In einer Ausführungsform des Kondenswassersammlers enthält das erfindungsgemäße Filzmaterial zudem Materialien, die Wärme oder Kälte speichern können, wie beispielsweise PCM oder Paraffin. Die niedrigen Temperaturen in der Nacht lassen den Wasserdampf am Morgen an der Filzfläche kondensieren. Die vom Tag gesammelte Restwärme steht den Pflanzen auch in der Nacht zur Verfügung. Zudem wird das erfindungsgemäße Filzmaterial optional beschichtet, so dass die Oberfläche IR-Strahlen reflektiert, um ein Aufheizen der Flächen am Tag zu vermeiden und hierdurch das Verdampfen von aufgenommenem Wasser zu verhindern. In einer alternativen Ausführungsform ist der Kondenzwassersammler aus dem erfindungsgemäßen Filzmaterial so ausgestaltet, dass die Filzfläche über Strukturen oder Öffnungen verfügt, aus denen heraus die Pflanzen wachsen, wobei diese Öffnungen möglichst klein gestaltet sind, um den Verlust und das Entweichen von Wasser zu vermeiden. Optimalerweise werden die Flächen aus dem erfindungsgemäßen Filzmaterial so aufgestellt, dass diese von der Luft bzw. dem Wind gleichmäßig umströmt werden und ausreichend vom Boden beabstandet sind, um den Pflanzenwuchs nicht zu behindern.

Besonders in Küsten in der Nähe von Seen oder anderen Wasserflächen in Waldoder Urwaldgebieten oder in Gebirgsnähe werden die Kondenswassersammler aus dem erfindungsgemäßen Filzmaterial, die mit Funktionsmaterialien wie PCM oder Paraffin beschlagen sind, effektiv eingesetzt. Dieses zeigt sich insbesondere in den Morgenstunden, wo die geringe Temperatur der erfindungsgemäßen Filzflächen auf eine hohe Luftfeuchtigkeit, Nebel oder Tau trifft und die Wärmespeichermedien die Kälte der Nacht speichern und hierdurch die Temperatur der erfindungsgemäßen Filzflächen länger kühl halten.

Weiterhin lassen sich die Kondenswassersammler aus den erfindungsgemäßen Filzflächen auch in Gewächshäusern oder bei Begrünungsflächen einsetzen oder können teilweise oder ganz direkt als Wachssubstrat verwendet oder mit diesem kombiniert werden.

Die Verwendung des erfindungsgemäßen Filzmaterials bzw. der Bauteile soll anhand der nachfolgenden Ausführungsformen und Ausführungsbeispiele weiter erläutert werden:
- Beim Einsatz in einer Oberfläche kann das Bauteil beispielsweise Teil eines Schienenbeinschützers oder Schutzhelms sein oder dieser daraus bestehen.
- Das Filzmaterial bzw. das Bauteil kann zur Belüftung oder Klimaregulierung von militärischen Schutzeinrichtungen, wie Schutzanzügen, verwendet werden. Beispielsweise bei Schutzanzügen von Jetpiloten, die unter Überdruck stehen, verhindert das Material das Eindringen von Wasser, sofern der Pilot im Schleudersitz aus dem Flugzeug aussteigen musste.
- Beim Einsatz in der Medizintechnik kann das Bauteil in Schienen, wie sie nach Knochenbrüchen angelegt werden, bzw. in schützenden Abdeckungen nach Verletzungen oder aufblasbaren Schienen für die Unfallerstversorgung eingesetzt werden. Durch die verbesserte Luftzirkulation wird der Heilungsprozess beschleunigt und sorgt zudem für einen erhöhten Tragekomfort. Bei der Anwendung ist dabei sowohl in der Veterinärmedizin als auch in der Humanmedizin möglich. In dieser Ausführungsform kann das Bauteil zudem mit einem Farbstoff versehen sein, welcher visualisiert, wie lange die Schiene getragen werden muss. Zudem können auch hydrochrome Farbstoffe eingesetzt werden, die anzeigen, wenn der Verband bzw. die Schiene mit Wasser in Berührung gekommen ist und deshalb ggf. ausgetauscht werden muss.
- Das Bauteil kann auch in oder für Möbelflächen eingesetzt werden, z.B. in den Liege- oder Sitzflächen von Kinderwagen, Kindertragen oder Babyautositzen oder als Überzug für Möbel oder andere gepolsterte Gegenstände. Hierdurch ist eine Belüftung bei gleichzeitigem Schutz vor Nässe bzw. Feuchtigkeit möglich. Ebenso ist eine Verwendung in waschbaren Matratzenauflagen oder zur Ummantelung von Materialien, die an sich keine Feuchtigkeit aufnehmen, denkbar. Beispielsweise eine Ummantelung von Luftmatratzen oder Nackenpolstern würde das Schwitzgefühl bei der Benutzung dieser Gegenstände verbessern.
- Das erfindungsgemäße Bauteil kann in der Bauindustrie eingesetzt werden, z.B. zur Belüftung von Gebäuden, bei gut schließenden Fenstern oder starker Isolation oder zur Vermeidung von Dampfsperren.
- Weitere Einsatzmöglichkeiten des erfindungsgemäßen Bauteils umfassen Abdeckungen, Kunststoffplanen und Gehäuse, wie Kabelbäume, Sicherungskästen, Scheinwerferboxen, bei denen durch das Bauteil die Ansammlung von Kondenswasser vermieden werden kann. Diese Vermeidung von Kondenswasser ist auch denkbar beim Einsatz des erfindungsgemäßen Bauteils in Geräten, PKW, Motorrädern, Wohnwagen, Containern, Laderäumen, Plakathalterungen, Zelten, Schlafsäcken, Schaukästen oder Gewächshäusern.
- Das Bauteil enthält optional auch zur Belüftung von Spezialbehältem genutzt, die zur Aufbewahrung von feuchtigkeits- oder flüssigkeitsempfindlichen Stoffen, wie Baustoffen (Zement, Kleber), Futtermitteln, Lebensmitteln oder medizinischen Produkten dienen.
- Das Bauteil kann neben dem Absorber Wirkstoffe enthalten und dadurch als Spezialfilter ausgestaltet sein, beispielsweise bei einer Anwendung im Schutzhelm, Staubsauger oder Staubmaske. Wird der Absorber im Bauteil mit Aktivkohle kombiniert, so wirkt die Aktivkohle gegen Gerüche, während das Bauteil gleichzeitig das Auftreffen von Flüssigkeiten auf die Elektronik verhindert.
- Das Bauteil kann so in einem Bekleidungsstück, einer Fläche oder einem Gegenstand angeordnet sein, dass es als Spezialventil dient und als Notwassersperrschicht oder Flüssigkeitssperre. Das Bauteil kann dabei mit elektronischen Sensoren gekoppelt werden, die z.B. den elektronischen Artikel abschalten bzw. eine bestimmte Steuerung herbeiführen, wenn das Bauteil mit Wasser oder Flüssigkeiten in Kontakt kommt.
- Das Bauteil kann so ausgestaltet sein, dass er als Stromgeber funktioniert: in dieser Ausführungsform ist eine Abdeckungsschicht aus Kupfer und die gegenüberliegende Abdeckungsschicht aus einem anderen Material z.B. Zink. Wenn der zwischen den Abdeckungsschichten liegende Absorber nass wird, entsteht ein elektrischer Strom, der z.B. für ein Signalgeber, wie eine Leuchtdiode oder andere elektronische Artikel verwendet werden kann oder eine Schaltung auslöst. Dieses macht es beispielsweise beim Einsatz in Kinderbekleidung oder Kinderschuhen möglich, dass diese, wenn sie nass wird, blinkt oder Töne von sich gibt. Dabei können die einzelnen Kammern des Bauteils wie Batterien hintereinander geschaltet werden.
- Zur Vorführung des erfindungsgemäßen Filzmaterials ist dieses in einem Testgerät integriert. Das Testgerät besteht z.B. aus einen zu einer Seite geöffneten Hohlkörper in den das erfindungsgemäße Bauteil eingesetzt ist. Im geöffneten Zustand ist es möglich durch das Bauteil, vergleichbar mit einer Trillerpfeffe Luft hindurch zu blasen. Wird das Bauteil angefeuchtet, verschließt sich dieser, was sich im Testgerät dadurch auswirkt, dass keine Luft mehr hindurch geblasen werden kann.
- Das erfindungsgemäße Filzmaterial kann als Filter eingesetzt werden, wobei das Superabsorberpolymer beispielsweise Wasser aus Treibstoffen bindet oder der Faseranteil zur Filterung von Staubpartikeln verwendet werden kann. Alternativ ist das erfindungsgemäße Filzmaterial zudem mit einem weiteren zur Filterung geeigneten Stoff, wie beispielsweise Aktivkohle oder einem Duftoder Wirkstoff ausgerüstet, der die Filterung begünstigt.
- Das erfindungsgemäße Filzmaterial kann als Dichtung, beispielsweise als O-Dichtung oder Flachringdichtung für Ventile oder als Unterlegescheibe eingesetzt werden. Diese hat den Vorteil, dass bestehende Dichtungen, welche nur einen statischen Zustand besitzen oder träge auf Wasser reagieren, gegen einfach herzustellende Dichtungsflächen ausgetauscht sind. Die einfachste Version einer luftdurchlässigen/ Druck ausgleichenden Dichtung besteht im Ausstanzen entsprechender O-Dichtungen/ Flächen aus dem erfindungsgemäßen Filzmaterial. Diese Dichtungselemente können zwischen zwei Flächen derart eingebracht sein, dass die Ausdehnung des Absorbers innerhalb des Filzgebildes zusätzlich zu der Begrenzung der Verbindungsfasern begrenzt wird. Je nach Anpressdruck lässt sich die Druckfestigkeit (bar), die Luftdurchlässigkeit und die Reaktionsgeschwindigkeit des Verschließens oder Öffnens steuern. Diese Eigenschaften werden auch durch den Grad des Verfilzens beeinflusst. Je nach dem, ob als Produkt eine sehr dichte Faserstruktur erreicht wird oder eher ein relativ loser Faserverbund der keine oder nur eine geringe Verdichtung des quellenden Absorbers ermöglicht.
- Das Filzmaterial ist zudem geeignet als Dichtung, beispielsweise als Ringdichtung, auch für Fenster oder Türen oder andere Anwendungen im Baubereich eingesetzt zu werden.
- Bei Verwendung des erfindungsgemäßen Filzmaterials als Dichtung ist dieses weiterhin als Abdichtungsmedium einsetzbar. Dabei wird das erfindungsgemäße Filzmaterial zwischen zwei Flächen eingebracht und füllt den Zwischenraum bei Quellung aus, so dass der Zwischenraum verschlossen wird. Hierzu werden beispielsweise dehnbare Fasern eingesetzt, die Filzfläche zwischen die abzudichtenden Flächen eingepresst oder ein Filzmaterial mit einem vordefinierten Volumenzuwachs verwendet.
- Das erfindungsgemäße Filzmaterial wird zudem in verschiedenen Anwendungen in der Verpackungsindustrie eingesetzt. So wird es als Einlage oder Bestandteil von Transportboxen oder Verpackungen für Backwaren verwendet. Derzeit werden Backwaren in offenen Verpackungen transportiert, die offen sind, um ein Entweichen von Feuchtigkeit zu ermöglichen. Dieses lässt jedoch keine optimal hygienische Verpackung der Backwaren zu, da die offenen Brotkisten Umwelteinflüssen ausgesetzt sind. Durch Verwendung des erfindungsgemäßen Filzeinsatzes kann ein "Schwitzen" der Backwaren verhindert werden bei gleichzeitiger hygienischer Verpackung. Da die absorberhaltige Schicht im erfindungsgemäßen Filzmaterial unverlierbar eingefilzt ist, ist auch bei Lebensmittelverpackungen sichergestellt, dass die Absorberpolymerpartikel nicht mit dem Lebensmittel in Berührung kommen. Eine weitere Verwendung in der Verpackungsindustrie stellt den Einsatz in Verpackungen für Tabakwaren dar, so dass in den Verpackungen eine optimale Feuchteregulierung ermöglicht wird.
- In einer weiteren Verwendung wird das erfindungsgemäße Filzmaterial für Drucksausgleichselemente in der Verpackungsindustrie eingesetzt. Die aus dem erfindungsgemäßen Filzmaterial hergestellten Drucksausgleichselemente werden beispielsweise auf die Verpackung aufgeschweißt oder anderweitig auf der Verpackung befestigt, so dass ein Luftaustausch der Verpackung mit der Umgebungsluft möglich ist, der Inhalt der Verpackung aber vor Wasser geschützt ist. Neben herkömmlichen Verpackungen finden diese Druckausgleichselemente beispielsweise im Deckel von Getränkebechern, Gährungsflaschen oder Behälterverschlüssen Anwendung.
- Bei einer weiteren Anwendung in der Verpackungsindustrie besteht die ganze Verpackung aus dem erfindungsgemäßen Filzmaterial oder eine Umhüllung oder ein Einsatz der Verpackung aus dem erfindungsgemäßen Filzmaterial. Dieses ist immer dann sinnvoll, wenn der Verpackungsinhalt sicher vor Kontakt mit Wasser geschützt werden soll, eine Luftzufuhr oder Belüftung jedoch erwünscht ist.
- Als feuchtigkeitsbindendes Futter oder in Polstermaterialien dient es der Feuchtigkeitsregulierung bzw. Entfeuchtung.
- Das erfindungsgemäße Filzmaterial bzw. die Bauteile können durch ihre luftfeuchtigkeitsbindende Wirkung auch als Funktionselement zwecks Entfeuchtung bzw. zur Klimaregulierung beispielsweise in Schutzhelmen oder Schutzanzügen. Schutzanzüge werden beispielsweise von Hochofenarbeitern, als Reinraumanzüge, als Motorradschutzbekleidung oder als Klima-Membranbekleidung getragen, wobei in den Anzügen, Zelten oder Isolierfolien entstehendes Kondenswasser nicht ausreichend entweichen kann. Durch das erfindungsgemäße Filzmaterial kann eine gute Luftzirkulation erreicht werden, wobei der Träger des Schutzanzuges rechtzeitig vor Nässe oder eintretenden Flüssigkeiten geschützt ist.
- Das erfindungsgemäße Filzmaterial kann so ausgebildet werden, dass es als Röhre zur Pflanzenbefeuchtung und Pflanzenbelüftung dient. Eine solche Röhre oder entsprechend anders geformtes Filzmaterial wird in das Erdreich oder Granulat gegeben und mit Flüssigkeit befüllt. Durch das zumindest teilweise durchlässige Filzmaterial wird Feuchtigkeit an die Pflanze abgegeben,
   wobei der Flüssigkeitspegel in der Bewässerungseinrichtung sinkt. Die nicht mehr mit Flüssigkeit gefüllten Bereiche ermöglichen aufgrund der Luftdurchlässigkeit nun eine Belüftung des Erdreichs bzw. Granulats. Insbesondere beim Einsatz von Pflanzgranulat, was häufig in geschlossenen Töpfen verwendet wird, kann so neben einer gleichzeitigen Befeuchtung eine verbesserte Belüftung der Pflanzenwurzeln erreicht werden.
- Aufgrund der Eignung zum Abtransport von Kondenswasser ist das erfindungsgemäße Filzmaterial geeignet für Schutzhüllen von elektronischen Geräten, wie insbesondere Labtops, Handys oder Kameras, in denen sich bei starken Temperaturschwankungen Kondenswasser sammelt. Ebenso kann das erfindungsgemäße Filzmaterial oder Bauteil hieraus als Einsatz im Gerät selbst eingesetzt werden, um Kondenswasser aus dem Gerät nach außen zu transportieren.
- Eine weitere Verwendung als Entfeuchtungseinsatz ist als Überzug für Handgriffe, beispielsweise Griffe von Taschen, Koffern, Werkzeug oder Fahrrädern denkbar, bei denen die Feuchtigkeit im Entstehen aufgenommen wird und zu einem späteren Zeitpunkt abgegeben wird, ohne dass sich der Handgriff unangenehm feucht anfasst oder durch die Feuchtigkeit zu einem Abrutschen kommt.
- In Scheibenwischerblätter wird das erfindungsgemäße Filzmaterial an Stelle oder zusätzlich zu der Gummilamelle verwendet, wobei es ebenfalls mit einem Wirkstoff versehen sein kann und diesen auf die Scheibe abgibt.
- Das erfindungsgemäße Filzmaterial findet weiter Verwendung als Dichtung oder Einsatz oder Dichtung für Stoma-Beutel oder deren Gewinde oder Steckverbindungen. Das erfindungsgemäße Filzmaterial ist hierzu beispielsweise mit Aktivkohle ausgerüstet, um die austretenden Gerüche zu filtern und gleichzeitig einen Druckausgleich zu ermöglichen.
- Die Verwendung des erfindungsgemäßen Filzmaterials zur Isolation gegen Kälte erfolgt in einer Vielzahl von Anwendungen. Beispielsweise sind dieses Unterwäsche, Schutzanzüge, Socken, herausnehmbare Innenschuhe, Schuhe, Bandagen, Immobilisierungsschienen, Korsette, Taucheranzüge, Taucherbrillen, Schutzbrillen, Mützen und Stirnbänder, Ummantelungen von Leitungen, Schlauchelemente, Ummantelung von elektronischen Geräten, wie z.B. Handys, Griffkomponenten, Kopfhörer, Lärmschutzauflagen, aufblasbare Jacken, Matratzen, Nackenpolster, Kissen, Bettdecken oder andere Umhüllungen oder Überzüge.
- Das erfindungsgemäße Filzmaterial wird im Baubereich beispielweise eingesetzt, um nach einem Wasserschaden das im Bau ausgetretene Wasser aufzunehmen. So entzieht das erfindungsgemäße Filzmaterial beispielsweise den Wänden das aufgenommene Wasser.
- Das erfindungsgemäße Filzmaterial wird zudem beispielsweise als flächige Abdeckung einer Öffnung verwendet. Hierbei wird es beispielsweise in oder auf einer Verpackung oder einem Gehäuse platziert. Auch ein Einsatz zur Flächenabdeckung von Öffnungen in Schutzhelmen ist eine mögliche Verwendung. Die flächige Abdeckung ist dabei entweder als abnehmbares Bauteil oder als fest am Gegenstand fixierte Abdeckung ausgebildet. Die flächige Abdeckung kann beispielsweise mit einem Material umhüllt sein, wie z.B. einem Laminat oder einer Folie und so am Gegenstand fixiert sein. In einer Ausführung ist die flächige Abdeckung einseitig mit einem Kunststoffmaterial oder einem zum Zeitpunkt der Verarbeitung fließfähigem Material überspritzt, das über den Filz hinausragende Seitenflächen bildet, die als Kontaktflächen mit dem abzudichtenden Gegenstand verbunden sind.
- Das erfindungsgemäße Filzmaterial ist ebenfalls als Bestandteil eines Entfeuchtungssystems einsetzbar. So wird es beispielsweise in einem Kondenstrockner verwendet, um eine schnelle Aufnahme und Bindung des Wassers und somit eine Entfeuchtung der Umgebung zu ermöglichen.
- Aus dem erfindungsgemäßen Material lassen sich zudem Kabelummantelungen, schlauchförmige Gebilde oder Schlauchummantelungen herstellen. Die Ummantelungen sind beispielsweise durch Umschlagen, Umwickeln oder Aufspritzen des Schlaumaterials auf ein erfindungsgemäßes textiles Filzmaterial zu erhalten. Wenn das erfindungsgemäße Filzmaterial beispielsweise die außenliegenden Schicht eines Feuerlöschschlauches ist, kann diese durch Perforation der wasserleitenden Medien, d.h. des inneren Schlauchs, mit Wasser benetzt oder getränkt werden, wodurch eine größere Hitzebeständigkeit des Löschschlauchs erreicht wird.
- In Staubfiltern oder Staubmasken lässt sich durch Verwendung des erfindungsgemäßen Filzmaterials eine Filterung eingestellt auf die zu filternde Partikelgröße realisieren. Durch Art der Fasern, Grad der Verdichtung und Dicke des Materials ist so eine selektive Filterung möglich. Ein entscheidender Vorteil beim Einsatz in diesen Schutzmasken oder Staubmasken ist die Feuchtigkeitsaufnahme, die ein Schwitzen innerhalb der Maske verhindert. Durch den angequollenen oder aufgequollenen Absorber werden die Staubpartikel fest am klebrigen Absorber gebunden. Nach dem Trocknen des Aborberpolymers lassen sich die Partikel beispielsweise durch Ausschlagen oder andere mechanische Behandlungen wieder ablösen.
- Wenn das erfindungsgemäße Filzmaterial im Inneren von Schutzhelmen, Schutzbrillen oder Schutzmasken eingesetzt wird, verhindert es durch die Feuchtigkeitsaufnahme ein Beschlagen der Sichtteile. Bei Schutzmasken werden zudem sensible Filtermedien vor Feuchtigkeit geschützt und somit die Funktionsdauer der Filtermedien erhöht.
- Die Feuchtigkeits- bzw. Wasseraufnahme des erfindungsgemäßen Filzmaterials wird ebenfalls genutzt, um Schweiß oder Feuchtigkeit von Gegenständen, die direkt mit dem Träger in Kontakt kommen, aufzunehmen. So dient das erfindungsgemäße Filzmaterial beispielsweise in Gürteln, Tragegurten, Rucksäcken, Schulterriemen, bei Uhrenarmbändern oder vergleichbaren Gegenständen dazu, den Schweiß aufzunehmen. Hierdurch wird verhindert, dass eine starke Durchnässung oder eine langanhaltende Feuchtigkeit gegeben ist. Auf diese Weise lassen sich die Tragesicherheit durch eine geringe Abrutschgefahr und der Tragekomfort erhöhen. Empfindliche Materialien, beispielsweise hochwertiges Leder, können auf diese Weise vor Feuchtigkeit und Schweiß geschützt werden, so dass sich die Lebensdauer erhöht.
- Eine weitere Verwendung des erfindungsgemäßen Filzmaterials stellt der Einsatz als Substratsmaterial für Pflanzen dar, wobei Blumentöpfe, Terrarien oder Pflanzgefäße mit dem Substratmaterial oder dem erfindungsgemäßen Filzmaterial ausgeschlagen werden könne.
- Das erfindungsgemäße Filzmaterial wird zudem in Kästen für Tuschfarben eingesetzt. Das Filzmaterial nimmt das im Kasten vorhandene Wasser auf und verhindert ein Auslaufen des Wassers. Durch die im Kasten gebundene Feuchtigkeit wird gleichzeitig ein Durchtrocknen der Farben verhindert.
- Das erfindungsgemäße Filzmaterial wird auch in Aufbewahrungsbehältem oder Aufbewahrungstaschen für nasse oder feuchte Gegenstände eingesetzt. Das erfindungsgemäße Filzmaterial entzieht durch die enthaltenen Absorberpolymere den Gegenständen die Feuchtigkeit und leitet diese bei entsprechender Ausgestaltung nach außen. Dieses ist z.B. für Aufbewahrungsbehälter von Sportbekleidung, Zahnpflegezubehör, Gebissboxen oder Zahnspangen ebenso relevant, wie für Reisetaschen, in denen nasse Handtücher oder andere nasse Gegenstände transportiert werden.
- Das erfindungsgemäße Filzmaterial wird als Bestandteil von Verpackungen bei Transport und Lagerung von Gegenständen, bei denen eine konstante Luftfeuchtigkeit gewünscht ist, verwendet. Dieses findet beispielsweise Anwendung bei Transport und Lagerung von PKW-Teilen oder Metallteilen, von Schüttgütern, wie Zement, Pulvern oder Baustoffen, von Kunstgegenständen oder bei der Holzabdeckung. Ebenso als Bestandteil in Füllerkappen gegen auslaufende Tinte und Austrocknen der Schreibfeder oder dem Austrocknen und Vertrocknen von Klebestiften oder Klebemitteln oder anderen feuchtigkeitsabgebenden Stoffen, die durch Abgabe der Feuchtigkeit ihre Qualität verlieren, wie Schuhpflegemitteln, Deos und anderen kosmetischen Gegenständen.
- In Gefrierverpackungen wird das erfindungsgemäße Filzmaterial als Einlage mit der Funktion eines Kühlakkus eingesetzt. Das Filzmaterial wird vor dem Einfrieren befeuchtet und speichert so im ungefrorenen Zustand Kälte, die an das Geriergut abgegeben werden kann. Alternativ bedient das erfindungsgemäße Filzmaterial zur Aufnahme von austretender Flüssigkeit und zur Vermeidung von Gefrierbrand.
- In einer Ausführungsform ist das erfindungsgemäße Filzmaterial einseitig mit einer ganz oder teilweise aus transparenten Fasern bestehenden Filzschicht versehen. Die transparenten Fasern erlauben den Durchtritt von Wärmestrahlung (IR-Strahlung) und so ein Erwärmen der absorberhaltigen Schicht. Die transparenten Fasern können dabei so gestaltet werden, dass die Wärmestrahlung nicht wieder aus dem Material reflektiert wird und so die Erwärmung der absorberhaltigen Schicht zusätzlich verstärkt wird.
- In einer weiteren Verwendung wird das erfindungsgemäße Filzmaterial als Einlage für Dunstabzugshauben verwendet. Hierdurch wird gezielt die im aufsteigenden Dunst enthaltene Feuchtigkeit aufgenommen. Das Material filtert zudem und lässt sich aufgrund der Waschbarkeit leicht reinigen.

Das erfindungsgemäße Filzmaterial lässt sich durch seine entfeuchtenden, befeuchtenden und klimaregulierenden Eigenschaften, sowie seine Sperrwirkung gegen Wasser und Flüssigkeiten als verschiedener Bestandteil oder als Material oder teilweises Material für eine Vielzahl von Gegenständen und Anwendungen einsetzen. Dieses umfasst beispielsweise Bügelbrettauflagen, Platinenauflagen, Auflagen auf elektronischen Bauteilen, Saugpads in Transportboxen oder Verpackungen, Innensohlen, auf Pelz oder Leder aufkaschiertes Filzmaterial für Innensohlen, Skistiefel, Skates, Schlittschuhe, Schuhe, Schuhbestandteile, Taucheranzüge, Schutzanzüge mit feuerfester Beschichtung, Schutzanzüge, Regenschirmständer, Schlafsäcke, Möbelbezüge, Stempelkissen, Papierproduktionen, Deponieabdeckungen, Begrünung von Flachdächern, Damm- oder Deichbau, Hochwasserschutz, Brandschutzsektor, Dentalpads, chirurgische Schwämme, Katzenklos, Transportboxen. Kindertransporteinheiten, Babytransporteinheiten, Tragegurte, Geotextilien, Agrartextilien, Pferdesattel, Schutzdecken, Staubsaugerbeutel, Kühlerhüllen für Getränkeflaschen, Kühlerhüllen für Lebensmittel, Tropfenfänger, Badematten, Regenschirmhüllen, Brillenflügel, Handauflageflächen, Abdeckhauben für Speisen, Tür- oder Fensterdichtungen oder Mülleimertütensaugpads.

Im Agrarbereich wird das erfindungsgemäße Filzmaterial beispielsweise so verwendet, dass Saatgut eingefilzt wird. Das Saatgut ist beispielsweise in Streifen eingearbeitet. Das mit dem Saatgut versehene erfindungsgemäße Filzmaterial wird dann in ein Wuchssubstrat oder auf ein Wuchssubstrat aufgebracht. Hierdurch lässt sich verhindern, dass das Saatgut verweht wird oder durch Tiere beschädigt wird. Das erfindungsgemäße Filzmaterial dient zudem als Wasserspeicher und erlaubt eine optimale Bewässerung. Optional können Dünger, Pestizide oder andere Wirkstoffe, die das Saatgut oder die daraus entstehenden Pflanzen positiv beeinflussen, mit in das Material eingebracht werden. Vorteilhafterweise sind in das erfindungsgemäße Filzmaterial in diesem Falle natürliche Fasern aus Stroh, Heu oder Moos und/oder Erde oder Wuchssubstrat eingefilzt.

Eine weitere bevorzugte Verwendung des erfindungsgemäßen Filzmaterials ist die Verwendung als Reinigungsgerät. Das erfindungsgemäße Filzmaterial ist dabei Bestandteil eines Wischgeräts, wie z.B. eines Wischmobs oder Reinigungstuchs, das optional mit einem Wirkstoff oder Reinigungsmittel versehen ist. Ist das Filzmaterial eine Lamelle in einem Wischgerät, so verhärtet sich dieses durch die Feuchtigkeit, hierdurch kommt es zu einem höheren Abrieb und ggf. gleichzeitige Abgabe von Wirkstoffen. Durch den höheren Abrieb tritt eine Polierwirkung auf, die insbesondere auf Parkettfußböden vorteilhaft ist. Bei Beaufschlagung mit einem Wirkstoff wird dieser Wirkstoff kontinuierlich und gleichmäßig abgegeben, so dass es zu einer gleichmäßigen Benetzung der zu reinigende Fläche kommt. Dieses stellt eine deutliche Verbesserung gegenüber herkömmlichen Wischgeräten her, die je nach aufgenommener Feuchtigkeitsmenge zu einer unterschiedlichen Verteilung des Reinigungsmittels führen.

Bei der Verwendung des erfindungsgemäßen Filzmaterials wird durch die Regulierung der Feuchtigkeit bei vielen Anwendungsbereichen eine Kältebrücke verhindert. Hierdurch wird bei Kälte eine bessere Isolation erreicht. Dieses ist insbesondere bei der Anwendung in Bekleidung, Schutzanzügen, wie z.B. Hochofen-Schutzanzügen, Baustoffen, Isolationsmaterialien oder Bodenbelägen sinnvoll.

Das erfindungsgemäße Filzmaterial hat den Vorteil, dass es eine gute Luftdurchlässigkeit aufweist und hierdurch eine Luftzirkulation und Belüftung ermöglicht wird. Da die Absorberpolymere fest in dem Fasergebilde eingebunden sind, lassen sich die erfindungsgemäßen Filzmaterialien aufgrund der mechanischen Belastbarkeit säubern bzw. abwaschen. Der Aufbau des erfindungsgemäßen Filzmaterials hat zudem den Vorteil, dass der glitschig oder gelartige Effekt, der bei der Verwendung von Superabsorbermaterialien in anderen Bereich entsteht, vermieden wird, da der Superabsorber als Kern fest in das Material eingebunden ist. Der glitschige oder gelartige Effekt entsteht bei anderen Materialien, da sich die verschiedenen Schichten des Absorbers bei Nässe gegeneinander verschieben lassen.

Das erfindungsgemäße Filzmaterial und die aus diesem Material hergestellten Bauteile weisen somit eine Reihe von Vorteilen auf, die andere vergleichbare Materialien nicht bieten. Bei dem erfindungsgemäßen Material lassen sich Eigenschaften, für die es sonst notwendig ist, mehrere Funktionsschichten über teilweise komplizierte Verbindungstechnologien miteinander zu verknüpfen, durch Nadelfilzen in einem Verarbeitungsprozess nur durch Einsatz verschiedener Schichten oder Bearbeitung der Schichten erreicht. Das erfindungsgemäße Material erlaubt eine einfache Handhabung und Weiterverarbeitung. So lässt es sich beispielsweise als Rollenwaren ausliefern und ist durch herkömmliche Techniken, wie z.B. Ausstanzung, Lasern oder Heißstanzen einfach zu konfektionieren. Das Material weist gegenüber herkömmlichen absorberhaltigen Materialien eine kontrollierte Volumenzunahme auf. Durch die Fasermatrix ist ein Austrittsschutz für die absorberhaltigen Materialien gegeben. Die Herstellung ist kostengünstig, da keine Spezialverfahren oder Spezialmaschinen außerdem erfindungsgemäßen Verfahren eingesetzt werden müssen.

Die Erfindung wird anhand der nachfolgenden Abbildungen beispielhaft beschrieben:

Es zeigt:
- Figur 1: Eine Ausführungsform des erfindungsgemäßen Filzmaterials vor dem Verfilzen.
- Figur 2:: Das Filzmaterial aus Figur 1 im verfilzten Zustand.
- Figur 3:: Eine weitere Ausführungsform des erfindungsgemäßen Filzmaterials vor dem Filzen.
- Figur 4:: Das Material aus Figur 3 nach dem Verfilzen.
- Figur 5:: Eine weitere Ausführungsform des erfindungsgemäßen Filzmaterials vor dem Verfilzen.
- Figur 6:: Das Filzmaterial aus Figur 5 nach dem Verfilzen.
- Figur 7:: Eine weitere Ausführungsform des erfindungsgemäßen Filzmaterials vor dem Verfilzen.
- Figur 8:: Eine Ausführung des erfindungsgemäßen Filzmaterials mit Perforationen bzw. Öffnungen.
- Figur 9:: Eine Ausführungsform des erfindungsgemäßen Filzmaterials mit Verdichtungselementen.
- Figur 10:: Ein Bauteil mit Randversiegelung aus dem Filzmaterial gemäß Figur 9.

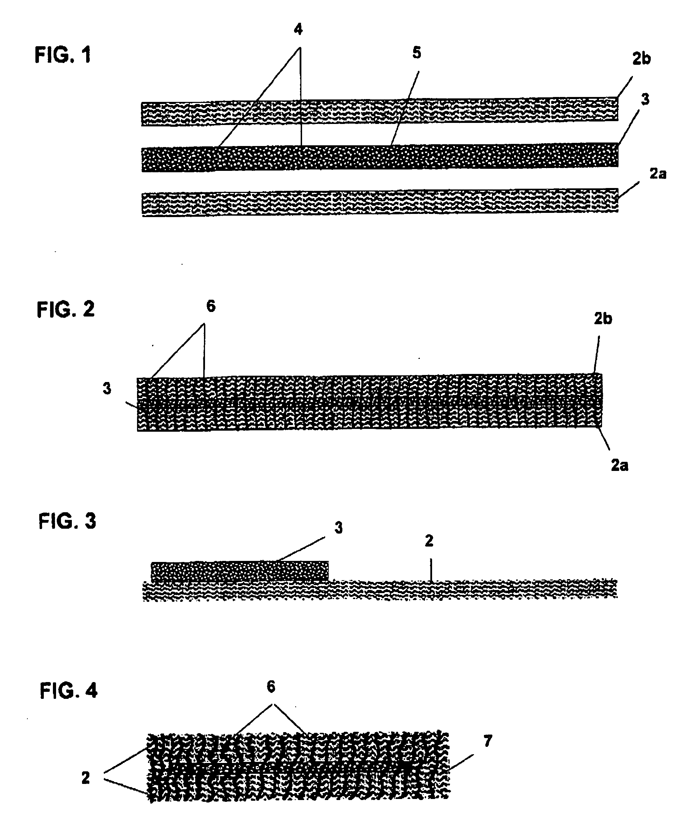

Figur 1 zeigt eine erste Filzschicht 2a, oberhalb der eine absorberhaltige Schicht 3 angeordnet ist. Die absorberhaltige Schicht besteht aus einem Absorber 4, der in einem Trägermaterial 5 eingebaut ist. Oberhalb der absorberhaltigen Schicht ist eine weitere Filzschicht 2b angeordnet.

Figur 2 zeigt das Filzmaterial aus Figur 2 nach dem Vernadeln. Die erste Filzschicht und die zweite Filzschicht 2b sind hier erkennbar über Verbindungsfasern 6 miteinander verbunden, die durch die absorberhaltige Schicht 3 hindurchführen. Der Absorber wird so nach oben und unten durch erste und zweite Filzschicht begrenzt, zu verschiedenen Seiten durch Verbindungsfasern 6.

Figur 3 zeigt eine weitere Ausführungsform des erfindungsgemäßen Filzmaterials vor dem Vernadeln. Die absorberhaltige Schicht 3 ist hier auf einer Filzschicht 2 angeordnet.

Figur 4 zeigt das Material aus Figur 3 nach dem Vernadeln. Die Filzschicht 2 wurde hier über die absorberhaltige Schicht 3 umgeschlagen und bildet so die Randversiegelung 7. Die Verbindungsfasern 6 sind durch die aus demselben Filzmaterial und derselben Filzschicht bestehende Filzschicht 2 durch die absorberhaltige Schicht getrieben.

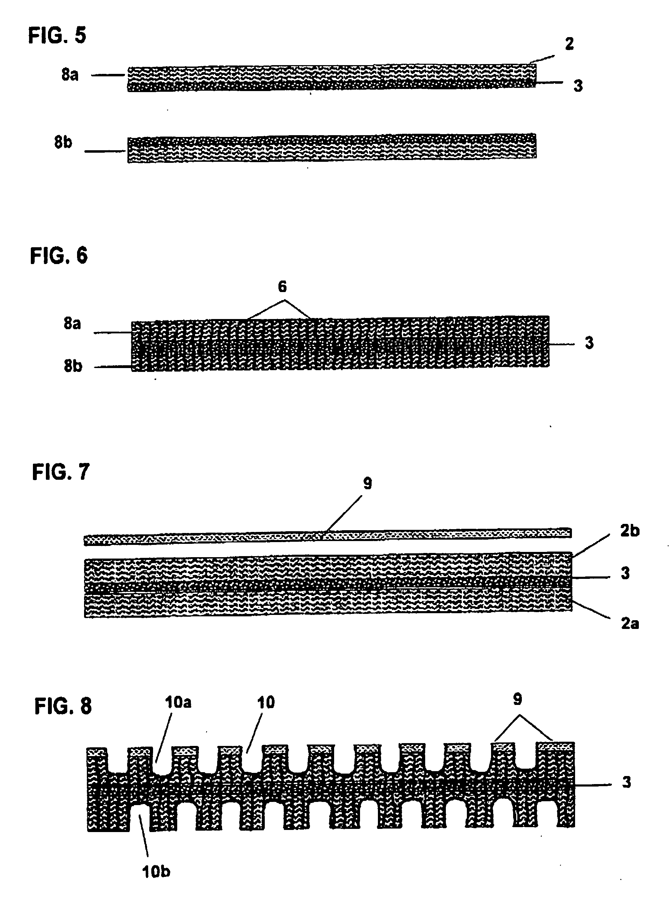

Figur 5 zeigt eine weitere Ausführungsform des erfindungsgemäßen Filzmaterials vor dem Vernadeln. Bei dieser Ausführungsform ist die absorberhaltige Schicht 3 Teil der Filzschicht 2 und bildet mit dieser gemeinsam die Verbundschicht 8a. Unterhalb der Verbundschicht 8a ist eine zweite Verbundschicht 8b angeordnet, wobei die beiden mit Absorber beschlagenen Seiten der Verbundschichten jeweils zueinander ausgerichtet sind.

Figur 6 zeigt die Verbundschichten 8a und 8b aus Figur 5 nach dem Vernadeln. Die absorberhaltigen Bereiche der Verbundschichten 8a und 8b bilden die mittig zwischen den Filzschichten eingeschlossene absorberhaltige Schicht 3.

Figur 7 zeigt eine weitere Ausführungsform des erfindungsgemäßen Filzmaterials, bei dem oberhalb der zweiten Filzschicht 2b eine Deckschicht 9 angeordnet ist. Die Deckschicht ist hier als wasser- und luftundurchlässige Kunststofffolie ausgebildet.

Figur 8 zeigt das Filzmaterial aus Figur 7 nach dem Vernadeln und Nachbehandeln. Die Deckschicht 9 ist ebenso wie die Filzschichten mit Perforationen bzw. Öffnungen 10 versehen. Die Perforationen 10 sind dabei jeweils nur so tief in die Filzschicht getrieben, dass oberhalb der absorberhaltigen Schicht 3 ein Teil der Filzschicht verbleibt. Bei der abgebildeten Variante sind die Öffnungen jeweils versetzt angeordnet, so dass die Öffnung 10a in der zweiten Filzschicht nicht der Öffnung 10b in der ersten Filzschicht gegenüberliegt.

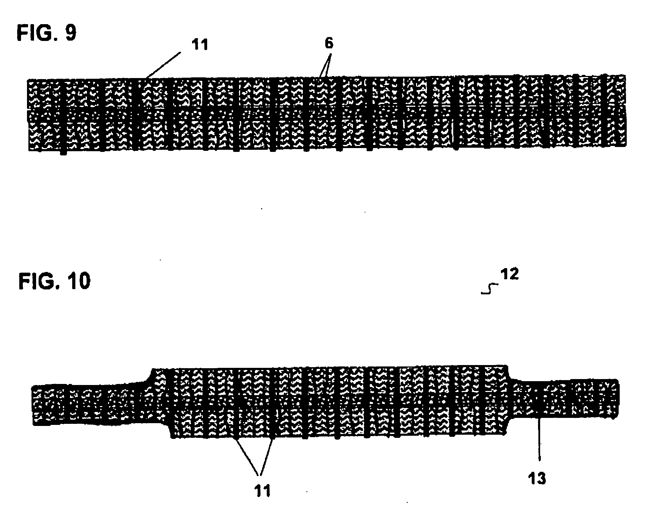

Figur 9 zeigt eine weitere Ausführungsform des erfindungsgemäßen Filzmaterials, das zu den Verbindungsfasern weitestgehend parallel laufende Sperrelemente 11 aufweist. Die Sperrelemente durchdringen das Filzmaterial streifenförmig und unterteilen dieses in Zellen. Hierdurch wird ebenfalls die Verdichtung des Absorbers unterstützt. Beim Austanzen oder Ausschneiden von Bauteilen aus dem Filzmaterial ist so zumindest teilweise eine Randversiegelung durch die Versteifungselemente 11 gegeben.

Figur 10 zeigt ein Bauteil 12, dass aus dem Filzmaterial in Figur 9 hergestellt ist. Das Bauteil weist neben den Sperrelementen 11 an beiden Seiten Verbindungselemente 13 auf, die zu einem Verbinden des Bauteils 12 mit dem zu belüftenden Objekt geeignet sind.

### Bezugszeichenliste

- 1: Filzmaterial
- 2: Filzschicht
- 3: absorberhaltige Schicht
- 4: Absorber
- 5: Trägermaterial
- 6: Verbindungsfasern
- 7: Randversiegelung
- 8: Verbundschicht
- 9: Deckschicht
- 10: Öffnungen
- 11: Sperrelement
- 12: Bauteil
- 13: Verbindungselemente

## Patentansprüche

1. Filzmaterial (1) mit Sperrfunktion zur Klimaregulierung umfassend mindestens zwei Filzschichten (2)
und mindestens in Teilbereichen mindestens eine flüssigkeitaufnehmende Schicht, wobei
- die mindestens eine flüssigkeitaufnehmende Schicht eine absorberhaltige Schicht (3) ist,
- die erste Schicht eine Filzschicht (2a) ist mit einer zumindest in Teilbereichen darauf angeordneten absorberhältigen Schicht und einer weiteren darauf angeordneten zweiten Filzschicht (2b),
- die mindestens zwei Filzschichten (2a) und die absorberhaltige Schicht (3) miteinander verfilzt sind,
- der Absorber in seiner dreidimensionalen Ausdehnung durch die Filzschichten und ggf. die Sperrelemente (11) begrenzt ist,
- das Filzmaterial (1) im trockenen, geöffneten Zustand zumindest teilweise luftdurchlässig ist und
- das Filzmaterial (1) bei Kontakt mit Flüssigkeit, Wasser oder Wasserdampf sich durch die Ausdehnung des Absorbers (4) verschließt, wobei der Flüssigkeitstransport durch das Filzmaterial (1) durch den gequollen Absorber (4) begrenzt oder gestoppt wird,
**dadurch gekennzeichnet, dass**
die Absorberschicht (3) ein Absorbervlies, ein Absorberpolymer, ein Absorber mit Trägermaterial oder Absorberfasern, und
der Absorber (4)
- ein Superabsorberpolymer oder
- ein quellfähiges Polymer, ausgewählt aus der Gruppe bestehend aus Polyacrylsäure, Polyacrylsäurecopolymeren, vernetzten Natriumpolyacrylat, Casein, Eiweiß und Thermoplast-Elastomer-Compositen oder
- eine Superabsorberpolymerfaser
ist,
der Absorber (4) in seiner dreidimensionalen Ausdehnung zumindest teilweise durch die Verbindungsfasern (6) zwischen den Filzschichten (2) begrenzt ist,
und das Filzmaterial (1) ein Nadelfilz ist.

2. Filzmaterial (1) gemäß Patentanspruch 1, **dadurch gekennzeichnet, dass** die Absorberschicht ein Vlies mit Absorberpolymeren ist.

3. Filzmaterial (1) gemäß einem der vorhergehenden Ansprüche, **dadurch gekennzeichnet, dass** die Filzschicht (2) aus synthetischen, halbsynthetischen, tierischen, mineralischen, metallischen, pflanzlichen, biologisch abbaubaren Fasern, Hybridfasern, Gummifasern oder einer Mischung hiervon besteht.

4. Filzmaterial (1) gemäß Anspruch 3, **dadurch gekennzeichnet, dass** die Filzschicht (2) zusätzlich Funktionsfasern enthält oder mit einem Funktionsmaterial vermischt ist, bevorzugt ein Phasenwechselmaterial (PCM) oder ein Formgedächnismaterial enthält.

5. Filzmaterial (1) gemäß einem der vorhergehenden Ansprüche, **dadurch gekennzeichnet, dass** die erste und die zweite Filzschicht (2) aus dem gleichen Material oder dass die die Filzschichten (2) aus unterschiedlichen Materialien bestehen.

6. Filzmaterial (1) gemäß einem der vorhergehenden Ansprüche, **dadurch gekennzeichnet, dass** die mindestens eine Filzschicht (2a) und die absorberhaltige Schicht (3) unddie zweite Filzschicht (2b) so miteinander vernadelt sind, dass das Filzmaterial (1) im trockenen Zustand luftdurchlässig ist und im nassen Zustand luftundurchlässig und wasserundurchlässig ist.

7. Filzmaterial (1) gemäß einem der vorhergehenden Ansprüche, **dadurch gekennzeichnet, dass** die absorberhaltige Schicht (3) zumindest teilweise aus wasserlöslichen oder lösemittellöslichen Fasern aufgebaut ist oder mit wasser- oder lösemittellöslichen Substanzen ausgerüstet ist, die mit dem Absorberpolymer verbunden sind oder das Absorberpolymer einschließen.

8. Filzmaterial (1) gemäß einem der vorhergehenden Ansprüche, **dadurch gekennzeichnet, dass** auf der ersten und/oder ggf. der zweiten Filzschicht (2b) eine Deckschicht (9) angeordnet ist oder die erste (2a) und/oder ggf. die zweite Filzschicht (2b) eine Oberflächenbehandlung aufweisen.

9. Filzmaterial (1) gemäß Anspruch 8, **dadurch gekennzeichnet, dass** die Deckschicht (9) Perforationen oder Öffnungen (10), Durchbrüche, Oberflächenstrukturen, Tunnelstrukturen, Vertiefungen, Soll-Knickstellen oder Soll-Bruchstellen und/oder Abtrennstellen aufweist.

10. Filzmaterial (1) gemäß einem der vorhergehenden Ansprüche **dadurch gekennzeichnet, dass** das Filzmaterial (1) mit Duftstoffen, Farbstoffen und/oder Wirkstoffen versehen ist.

11. Filzmaterial (1) gemäß einem der vorhergehenden Ansprüche **dadurch gekennzeichnet, dass** das Filzmaterial (1) zusätzlich
- Formteile, bevorzugt Kunststoff oder Filzformteile, enthält, die mit mindestens einer Filzschicht (2) vefilzt sind oder im Filzmaterial eingefilzt sind und/oder
- Sperrelemente (11), bevorzugt durchspritzte Sperrelemente in Richtung der Verbindungsfasern,
enthält.

12. Filzmaterial (1) gemäß einem der vorhergehenden Ansprüche **dadurch gekennzeichnet, dass** das Filzmaterial (1) die Oberseite und die Unterseite des erfindungsgemäßen Filzmaterials unterschiedliche Eigenschaften haben.

13. Verfahren zur Herstellung eines Filzmaterials (1) umfassend mindestens zwei Filzschichten (2) und zumindest teilweise mindestens eine absorberhaltige Schicht (3),
wobei der Absorber bei Kontakt mit Flüssigkeit, Wasser oder Wasserdampf aufquillt, **dadurch gekennzeichnet, dass**
(a.1)- die mindestens eine absorberhaltige Schicht (3) auf eine Filzschicht (2a) oder zwischen zwei Filzschichten platziert wird und
(a.2)- die Filzschichten (2) und die absorberhaltige Schicht (3) mit Nadeln miteinander verfilzt werden wobei die absorberhaltige Schicht (3) ein Absorbervlies ist, wobei die Fasern der einen Filzschicht die weitere Filzschicht durchdringen und Verbindungsfasern bilden
oder
(b.1)- eine Schicht mit Vorprodukten des Absorberpolymers auf eine Filzschicht (2a) oder zwischen zwei Filzschichten platziert wird und
(b.2)- die Filzschichten und die vorproduktehaltige Schicht mit Nadeln miteinander verfilzt werden und die Polymerisation zum Absorberpolymer während oder nach dem Filzvorgang abläuft und beendet wird,
wobei die Fasern der einen Filzschicht die weitere Filzschicht durchdringen und Verbindungsfasern bilden.

14. Verfahren gemäß Patentanspruch 13, **dadurch gekennzeichnet, dass** die absorberhaltige Schicht (3) aus wasserlöslichen oder lösemittellöslichen Fasern aufgebaut ist, die mit dem Absorberpolymer verbunden sind oder das Absorberpolymer einschließt.

15. Verfahren gemäß einem der Patentansprüche 13 oder 14, **dadurch gekennzeichnet, dass** der Absorber (4)
- ein Superpolymerabsorber oder
- ein quellfähiges Polymer, ausgewählt aus der Gruppe bestehend aus Polyacrylsäure, Polyacrylsäurecopolymeren, vernetzten Natriumpolyacrylat, Casein, Eiweiß und Thermoplast-Elastomer-Compositen oder
- eine Superabsorberpolymerfaser
ist.

16. Verfahren nach einem der Patentansprüche 13 bis 15, **dadurch gekennzeichnet, dass** die Filzschicht (2) aus synthetischen, halbsynthetischen, tierischen, mineralischen, metallischen, pflanzlichen, biologisch abbaubaren Faser, Gummifasern, Hybridfasern oder einer Mischung hiervon besteht und/oder die erste und zweite Filzschicht (2) aus dem gleichen Material oder unterschiedlichen Materialien bestehen.

17. Verfahren gemäß einem der Patentansprüche 13 bis 16, **dadurch gekennzeichnet, dass** nach dem Vernadeln zumindest teilweise eine Deckschicht (9) auf die erste und/oder zweite Filzschicht (2) aufgebracht wird und ggf. die Deckschicht (9) beim oder nach dem Aufbringen mit Perforationen und/oder Öffnungen (10) versehen wird.

18. Verfahren gemäß einem der Patentanspruch 13 bis 17, **dadurch gekennzeichnet, dass** in einem weiteren Verfahrensschritt ein Nassfilzvorgang durchgeführt wird.

19. Verfahren gemäß einem der Patentansprüche 13 bis 18, **dadurch gekennzeichnet, dass** beim Nadelfilzen zumindest teilweise Hohlnadein eingesetzt werden, durch die Substanzen beim Filzvorgang in das Material eingebracht werden.

20. Verfahren gemäß einem der Patentansprüche 13 bis 19, **dadurch gekennzeichnet, dass** das Filzmaterial (1) zumindest teilweise eine Oberflächenbehandlung aufweist.

21. Verfahren gemäß einem der Patentansprüche 13 bis 20, **dadurch gekennzeichnet, dass** die absorberhaltige Schicht (3) vor der Platzierung auf der Filzschicht (2) gequollen oder angequollen ist und im gequollenen oder angequollenen Zustand überfilzt wird.

22. Verfahren gemäß einem der Patentansprüche 13 bis 21, **dadurch gekennzeichnet, dass** Formteile auf die Filzschicht (2) und/oder absorberhaltige Schicht (3) positioniert werden, die beim Vernadeln mit mindestens einer der Schichten verfilzt werden.

23. Verfahren gemäß einem der Patentansprüche 13 bis 22, **dadurch gekennzeichnet, dass** das Filzmaterial (1) zur Erzeugung von Sperrelemente (11) mit Kunststoff durchspritzt wird, bevorzugt in Richtung der Verbindungsfasern (6) durchspritzt.

24. Verfahren gemäß einem der Patentansprüche 13 bis 23 **dadurch gekennzeichnet, dass** ein Filzmaterial (1) gemäß einem der Patentansprüche 1 bis 13 hergestellt wird.

25. Bauteil (12) aus Filzmaterial (1) gemäß einem der Ansprüche 1 bis 12 oder aus einem Filzmaterial (1) hergestellt nach einem der Ansprüche 13 bis 24 **dadurch gekennzeichnet, dass** das Bauteil (12) eine Randversiegelung (7) aufweist.

26. Verwendung eines Filzmaterials gemäß einem der Patentansprüche 1 bis 12 oder eines Materials hergestellt nach einem Verfahren gemäß einem der Ansprüche 13 bis 24 oder eines Bauteils gemäß Anspruch 25 zur Belüftung, Entfeuchtung, Befeuchtung, Abdichtung und/oder Klimaregulierung von textilen oder nicht-textilen Gegenständen.

27. Verwendung gemäß Patentanspruch 26 als Belüftungseinsatz oder Belüftungsfläche und/oder Entfeuchtungseinsatz oder Befeuchtungseinsatz für Textilien, Bekleidung, Schuhe, Schuhinnensohlen, Einlegesohlen, Futter, Polstermaterialien, Überzüge, Decken, Abdeckungen, Zelten, Schutzhelme, Schutzanzüge, Schutzhüllen, Verbände, orthopädische Artikel, Prothesen, Behälter, Gehäuse oder Gebinde.

28. Verwendung gemäß Patentanspruch 26 als technische Filter, Dichtungen, Dichtungsstränge, Saugpads, Schwämme, Verbandsmaterialien, Kondenswassersammler oder Reinigungsgerät.

29. Verwendung gemäß einem der Patentansprüche 26 oder 28 als Dichtung, Ringdichtung, O-Ring, Unterlegscheibe Entfeuchtungseinsatz in Bekleidung, Verpackungen oder Gehäusen oder Bauteil von Gehäusen.

## Claims

1. Felt material (1) having a barrier function for climatic regulation, comprising at least two felt layers (2)
and at least one fluid-absorbing layer at least in partial areas,
wherein
- the at least one fluid-absorbing layer is an absorber-containing layer (3),
- the first layer is a felt layer (2a) which has an absorber-containing layer arranged thereupon at least in partial areas and a further second felt layer (2b) arranged thereupon,
- the at least two felt layers (2a) and the absorber-containing layer (3) are felted to one another,
- the absorber is limited in its three-dimensional expansion by the felt layers and optionally the blocking elements (11),
- the felt material (1) is at least partially air-permeable in the dry, open state, and
- the felt material (1) is closed when it comes into contact with fluid, water or water vapour by the expansion of the absorber (4), the fluid transport through the felt material (1) being limited or stopped by the swollen absorber (4), **characterised in that**
the absorber layer (3) is an absorber nonwoven material, an absorber polymer, an absorber with a carrier material or absorber fibres, and
the absorber (4) is
- a superabsorbent polymer or
- a swellable polymer, selected from the group consisting of polyacrylic acid, polyacrylic acid copolymers, crosslinked sodium polyacrylate, casein, albumen and thermoplastic elastomer composites or
- a superabsorbent polymer fibre,
the absorber (4) is limited in its three-dimensional expansion at least partially by the connecting fibres (6) between the felt layers (2),
and the felt material (1) is a needle felt.

2. Felt material (1) according to Claim 1, **characterised in that** the absorber layer is a nonwoven material with absorber polymers.

3. Felt material (1) according to one of the preceding claims, **characterised in that** the felt layer (2) is made of synthetic, semi-synthetic, animal, mineral, metallic, vegetable, biodegradable fibres, hybrid fibres, rubber fibres or a mixture thereof.

4. Felt material (1) according to Claim 3, **characterised in that** the felt layer (2) additionally contains functional fibres or is mixed with a functional material, preferably a phase change material (PCM) or a shape memory material.

5. Felt material (1) according to one of the preceding claims, **characterised in that** the first and the second felt layer (2) are made of the same material or **in that** the felt layers (2) are made of different materials.

6. Felt material (1) according to one of the preceding claims, **characterised in that** the at least one felt layer (2a) and the absorber-containing layer (3) and the second felt layer (2b) are needled to one another such that the felt material (1) is air-permeable in the dry state and is air-impermeable and water-impermeable in the wet state.

7. Felt material (1) according to one of the preceding claims, **characterised in that** the absorber-containing layer (3) is constructed at least partially from water-soluble or solvent-soluble fibres or is equipped with water-soluble or solvent-soluble substances, which are connected to the absorber polymer or enclose the absorber polymer.

8. Felt material (1) according to one of the preceding claims, **characterised in that** a covering layer (9) is arranged on the first and/or optionally the second felt layer (2b) or the first (2a) and/or optionally the second felt layer (2b) have a surface treatment.

9. Felt material (1) according to Claim 8, **characterised in that** the covering layer (9) has perforations or openings (10), apertures, surface structures, tunnel structures, depressions, designated bending locations or designated breaking locations and/or separating locations.

10. Felt material (1) according to one of the preceding claims, **characterised in that** the felt material (1) is provided with fragrances, dyes and/or active substances.

11. Felt material (1) according to one of the preceding claims, **characterised in that** the felt material (1) additionally contains
- shaped parts, preferably plastic or felt shaped parts, which are felted to at least one felt layer (2) or are felted in the felt material and/or
- blocking elements (11), preferably blocking elements injected through in the direction of the connecting fibres.

12. Felt material (1) according to one of the preceding claims, **characterised in that** the felt material (1) the top side and the bottom side of the felt material according to the invention have different properties.

13. Method for producing a felt material (1) comprising at least two felt layers (2) and at least partially at least one absorber-containing layer (3),
wherein the absorber swells when it comes into contact with fluid, water or water vapour,
**characterised in that**
(a.1)- the at least one absorber-containing layer (3) is placed on one felt layer (2a) or between two felt layers, and
(a.2)- the felt layers (2) and the absorber-containing layer (3) are felted to one another with needles, wherein the absorber-containing layer (3) is an absorber nonwoven material,
wherein the fibres of the one felt layer penetrate through the other felt layer and form connecting fibres,
or
(b.1) one layer with precursors of the absorber polymer is placed on one felt layer (2a) or between two felt layers, and
(b.2)- the felt layers and precursor-containing layer are felted to one another with needles and the polymerisation to form the absorber polymer takes place and is completed during or after the felting process,
wherein the fibres of the one felt layer penetrate through the other felt layer and form connecting fibres.

14. Method according to Claim 13, **characterised in that** the absorber-containing layer (3) is constructed from water-soluble or solvent-soluble fibres, which are connected to the absorber polymer or enclose the absorber polymer.

15. Method according to one of Claims 13 or 14, **characterised in that** the absorber (4) is
- a superpolymer absorber or
- a swellable polymer, selected from the group consisting of polyacrylic acid, polyacrylic acid copolymers, crosslinked sodium polyacrylate, casein, albumen and thermoplastic elastomer composites or
- a superabsorbent polymer fibre.

16. Method according to one of Claims 13 to 15, **characterised in that** the felt layer (2) is made of synthetic, semi-synthetic, animal, mineral, metallic, vegetable, biodegradable fibres, rubber fibres, hybrid fibres or a mixture thereof and/or the first and second felt layer (2) are made of the same material or different materials.

17. Method according to one of Claims 13 to 16, **characterised in that** after needling, a covering layer (9) is applied at least partially to the first and/or second felt layer (2) and optionally the covering layer (9) is provided with perforations and/or openings (10) during or after the application.

18. Method according to one of Claims 13 to 17, **characterised in that** in a further method step a wet-felting process is carried out.

19. Method according to one of Claims 13 to 18, **characterised in that** hollow needles are employed at least partially during the needle-felting, through which needles substances can be introduced into the material during the felting process.

20. Method according to one of Claims 13 to 19, **characterised in that** the felt material (1) has at least partially a surface treatment.

21. Method according to one of Claims 13 to 20, **characterised in that** the absorber-containing layer (3) is swollen or partially swollen before it is placed on the felt layer (2) and it is overfelted in the swollen or partially swollen state.

22. Method according to one of Claims 13 to 21, **characterised in that** shaped parts are positioned on the felt layer (2) and/or absorber-containing layer (3), which are felted during the needling to at least one of the layers.

23. Method according to one of Claims 13 to 22, **characterised in that** plastic material is injected through the felt material (1), preferably in the direction of the connecting fibres (6), in order to produce blocking elements (11).

24. Method according to one of Claims 13 to 23, **characterised in that** a felt material (1) according to one of Claims 1 to 13 is produced.

25. Component (12) made of felt material (1) according to one of Claims 1 to 12 or produced from a felt material (1) according to one of Claims 13 to 24, **characterised in that** the component (12) has an edge seal (7).

26. Use of a felt material according to one of Claims 1 to 12 or of a material produced by a method according to one of Claims 13 to 24 or of a component according to Claim 25 for ventilating, dehumidifying, humidifying, sealing and/or climatic regulation of textile or non-textile objects.

27. Use according to Claim 26 as a ventilation insert or ventilation surface and/or dehumidification insert or humidification insert for textiles, clothing, shoes, inner soles of shoes, insert soles, lining, cushion materials, coverings, blankets, covers, tents, safety helmets, protective suits, protective covers, bandages, orthopaedic articles, prostheses, containers, housings or packages.

28. Use according to Claim 26 as technical filters, seals, sealing strips, absorbent pads, sponges, bandage materials, condensation water collectors or cleaning apparatus.

29. Use according to one of Claims 26 or 28 as a seal, ring seal, O-ring, washer, dehumidification insert in clothing, packaging or housings or component of housings.

## Revendications

1. Matériau du type feutre (1) doté d'une fonction de blocage en vue d'une régulation climatique, comprenant au moins deux couches (2) de feutre et au moins une couche recevant des liquides, au moins dans des zones partielles,
- la couche, au moins unique, recevant des liquides étant une couche (3) renfermant un absorbant, la première couche étant une couche de feutre (2a), comportant une couche renfermant un absorbant disposée sur celle-ci, au moins dans des zones partielles, et une autre, deuxième, couche de feutre (2b) disposée sur celle-ci,
- les au moins deux couches de feutre (2) et la couche renfermant un absorbant (3) étant feutrées les unes avec les autres,
- l'absorbant étant limité dans son expansion tridimensionnelle par les couches de feutre et le cas échéant par les éléments de blocage (11),
- le matériau du type feutre (1) laissant passer l'air au moins partiellement lorsqu'il est à l'état sec, ouvert,
- le matériau du type feutre (1) se fermant lors du contact avec un liquidé, de l'eau ou de la vapeur d'eau, du fait de l'expansion de l'absorbant (4), le passage de liquides à travers le matériau du type feutre (1) étant limité ou arrêté du fait du gonflement de l'absorbant (4),
**caractérisé en ce que**
la couche (3) d'absorbant est une nappe absorbante, un polymère absorbant, un absorbant comportant un matériau de support, ou des fibres absorbantes, et
l'absorbant (4) étant
- un polymère superabsorbant ou
- un polymère apte à gonfler, choisi dans le groupe que constituent l'acide polyacrylique, les copolymères de l'acide polyacrylique, des polyacrylates de sodium réticulés, la caséine, l'albumine et les composites thermoplastique-élastomère ou une fibre de polymère superabsorbent,
- l'absorbant (4) étant limité au moins partiellement dans son expansion tridimensionnelle par les fibres de liaison (6) entre les couches de feutre (2) et **en ce que** le matériau du type feutre (1) est un feutre aiguilleté.

2. Matériau du type feutre (1) selon la revendication 1 **caractérisé en ce que** la couche d'absorbant est une nappe comportant des polymères absorbants.

3. Matériau du type feutre (1) selon une des revendications qui précèdent, **caractérisé en ce que** la couche de feutre (2) est constituée de libres synthétiques, semi-synthétiques, animales, minérales, métalliques, végétales décomposables biologiquement, de fibres hybrides, de fibres de caoutchouc ou d'un mélange de celles-ci.

4. Matériau du type feutre (1) selon la revendication 3 **caractérisé en ce que** la couche de feutre (2) contient en outre des fibres fonctionnelles, ou bien est mélangée à un matériau fonctionnel, de préférence un matériau apte à changer de phase (PCM) ou un matériau à mémoire de forme.

5. Matériau du type feutre (1) selon l'une des revendication qui précèdent, **caractérisé en ce que** la première et la deuxième couches de feutre (2) sont constituées du même matériau ou **en ce que** les couches de feutre (2) sont constituées de matériaux différents.

6. Matériau du type feutre (1) selon l'une des revendications qui précèdent, **caractérisé en ce que** la couche de feutre (2a) au moins unique et la couche renfermant l'absorbant (3) sont aiguilletées ensemble de telle manière que le matériau du type feutre (1) laisse passer l'air à l'état sec et est étanche à l'air et étanche à l'eau à l'état mouillé.

7. Matériau du type feutre (1) selon l'une des revendications qui précèdent, **caractérisé en ce que** la couche renfermant l'absorbant (3) est constituée, au moins partiellement, de fibres solubles dans l'eau ou de libres solubles dans un solvant, ou est dotée de substances solubles dans l'eau ou de substances solubles dans un solvant, qui sont liées au polymère absorbant ou qui enferment le polymère absorbant.

8. Matériau du type feutre (1) selon l'une des revendications qui précèdent, **caractérisé en ce qu'**une couche de couverture est disposée sur la première couche de feutre et/ou le cas échéant sur la deuxième couche de feutre (2b) ou **en ce que** la première couche de feutre (2a) et/ou le cas échéant la deuxième couche de feutre (2b) présentent un traitement de surface.

9. Matériau du type feutre (1) selon la revendication 8 **caractérisé en ce que** la couche de couverture (9) présente des perforations ou des ouvertures (10), des déchirures, des structures superficielles, des structures en forme de tunnel, des creux, des emplacements de pliage prévus, ou des emplacements de rupture prévus et/ou des emplacements de séparation.

10. Matériau du type feutre (1) selon l'une des revendications qui précèdent, **caractérisé en ce que** le matériau du type feutre (1) est doté de matières odorantes, de matières colorantes et/ou de matières actives.

11. Matériau du type feutre (1) selon l'une des revendications qui précèdent, **caractérisé en ce que** le matériau du type feutre (1) contient en outre
- des pièces de forme, de préférence des pièces de forme en matière de synthèse ou des pièces de forme en feutre, qui sont munies d'au moins une couche de feutre (2), ou qui sont incorporées par feutrage dans le matériau du type feutre et/ou des éléments de blocage (11) de préférence des éléments de blocage injectés dans la direction des fibres de liaison.

12. Matériau du type feutre (1) selon l'une des revendications qui précèdent, **caractérisé en ce que** la face supérieure et la face inférieure du matériau du type feutre selon l'invention ont des propriétés différentes.

13. Procède de fabrication d'un matériau du type feutre (1) comprenant au moins deux couches de feutre (2) et au moins partiellement au moins une couche (3) contenant un absorbant.
l'absorbant gonflant au contact d'un liquide, d'eau ou de vapeur d'eau, **caractérisé en ce que**
(a.1) - la couche (3) au minimum unique, renfermant un absorbant est mise en place sur une couche de feutre (2a) ou entre deux couches de feutre et
(a.2) - les couches de feutre (2) et la couche (3) renfermant un absorbant sont feutrées ensemble au moyen d'aiguilles, la couche (3) renfermant un absorbant étant une nappe d'absorbant, les fibres d'une couche de feutre traversant la couche de feutre suivante et formant des fibres de liaison,
ou
(b.1) - une couche comportant les produits de base du polymère absorbant est mise en place sur une couche de feutre (2a) ou entre deux couches de feutre et
(b.2) - les couches de feutre et la couche contenant les produits de base sont feutrées ensemble au moyen d'aiguilles et la polymérisation conduisant au polymère absorbant se déroule et se termine pendant ou après l'opération de feutrage,
les libres d'une couche de feutre traversant la couche de feutre suivante et formant des fibres de liaison.

14. Procédé selon la revendication 13 **caractérisé en ce que** la couche (3) renfermant un absorbant est constituée de fibres solubles dans l'eau ou de fibres solubles dans un solvant, qui sont liées au polymère absorbant ou qui renferment le polymère absorbant.

15. . Procédé selon l'une des revendications 13 ou 14, **caractérisé en ce que** l'absorbant (4)
- est un polymère du type superabsorbant ou
- est un polymère susceptible de gonfler, choisi dans le groupe que constituent l'acide polyacrylique, les copolymères de l'acide polyacrylique, de polyacrylate de sodium réticulé, la caséine, l'albumine et les composites thermoplastique-élastomère, ou
- est une fibre de polymère du type superabsorbant.

16. Procédé selon l'une des revendications 13 à 15, **caractérisé en ce que** la couche de feutre (2) est constituée de fibres synthétiques, semi-synthétiques, animales. minérales, métalliques, végétales, décomposables biologiquement, de fibres de caoutchouc, de fibres hybrides ou d'un mélange de celles-ci, et/ou **en ce que** la première et la deuxième couches de feutre (2) sont constituées du même matériau ou de matériaux différents.

17. Procédé selon l'une des revendications 13 à 16, **caractérisé en ce qu'**après l'aiguilletage une couche de couverture (9) est disposée au moins partiellement sur la première couche de feutre et/ou sur la deuxième couche de feutre (2), et **en ce que**, le cas échéant, la couche de couverture (9) est munie de perforations et/ou d'ouvertures (10) avant ou après la mise en place.

18. Procédé selon l'une des revendications 13 à 17, **caractérisé en ce que** lors d'un pas de procédé ultérieur une opération de feutrage humide est exécutée.

19. Procédé selon l'une des revendications 13 à 18, **caractérisé en ce que** lors de l'aiguilletage on utilise au moins partiellement des aiguilles creuses par lesquelles les substances sont introduites dans le matériau lors de l'opération de feutrage.

20. Procédé selon l'une des revendications 13 à 19, **caractérisé en ce que** le matériau du type feutre (1) présente au moins partiellement un traitement de surface.

21. Procédé selon l'une des revendications 13 à 20, **caractérisé en ce que** la couche (3) contenant un absorbant se trouve, avant la mise en place sur la couche de feutre (2), à l'état gonflé ou à l'état de gonflement amorcé et est feutrée sur celle-ci à l'état gonflé ou à l'état de gonflement amorcé.

22. Procédé selon l'une des revendications 13 à 21, **caractérisé en ce que** des pièces de forme sont disposées sur la couche de feutre (2) et/ou sur la couche (3) renfermant un absorbant, lesquelles pièces de forme sont feutrées avec au moins une des couches lors de l'aiguilletage.

23. Procédé selon l'une des revendications 13 à 22, **caractérisé en ce que** le matériau de type feutre (1) subit une injection traversante de matière de synthèse en vue de la réalisation d'éléments de blocage (11), de préférence dans la direction des fibres de liaison (6).

24. Procédé selon l'une des revendications 13 à 23, **caractérisé en ce qu'**un matériau du type feutre (1) est réalisé selon une des revendications 1 à 13.

25. Pièce constitutive (12) en matériau du type feutre (1) selon l'une des revendications 1 à 12, ou bien fabriquée selon une des revendications 13 à 24 à partir d'un matériau du type feutre (1), **caractérisé en ce que** la pièce constitutive (12) présente un scellage (7) sur ses bords.

26. Utilisation d'un matériau du type feutre selon l'une des revendications 1 à 12, ou d'un matériau fabriqué d'après un procédé selon une des revendications 13 à 24, ou d'une pièce constitutive selon la revendication 25 en vue de l'aération, de la déshumidification, de l'humidification, de l'étanchéification et/ou de la régulation climatique d'objets textiles et non textiles.

27. Utilisation selon la revendication 26 en tant qu'insert d'aération ou en tant que surface d'aération et/ou en tant qu'insert de déshumidification ou en tant qu'insert d'humidification pour des textiles, des vêtements, des chaussures, des semelles intérieures de chaussures, des semelles à insérer, des doublures, des matériaux pour des pièces d'ameublement capitonnées, des revêtements, des couvertures, des caches, des tentes, des casques de protection, des vêtements de protection, des capuches de protection, des bandages, des articles orthopédiques, des prosthèses, des récipients, des boîtiers ou des emballages.

28. Utilisation selon la revendication 26 en tant que filtres techniques, joints, cordons d'étanchéité, tampons absorbants, éponges, matériaux pour bandages, collecteurs d'eau de condensation ou appareils de nettoyage.

29. Utilisation selon une des revendications 26 ou 28 en tant que joints, joints annulaires, joints toriques, rondelles d'appui, inserts de déshumidification, boîtiers ou pièces constitutives de boîtiers.
